(19) Europäisches Patentamt / European Patent Office / Office européen des brevets

(11) **EP 4 106 513 B1**

(12) **EUROPEAN PATENT SPECIFICATION**

(45) Date of publication and mention of the grant of the patent:
**20.12.2023 Bulletin 2023/51**

(21) Application number: **21713783.5**

(22) Date of filing: **20.02.2021**

(51) International Patent Classification (IPC):
*A01G 9/14* (2006.01)   *A01G 13/02* (2006.01)
*A61F 13/532* (2006.01)   *A61L 15/60* (2006.01)
*A61L 15/62* (2006.01)   *B32B 27/32* (2006.01)

(52) Cooperative Patent Classification (CPC):
(C-Sets available)
**B32B 5/26; A61F 13/51401; A61F 13/51405; A61L 15/24; A61L 15/60;** A01F 25/13;
B32B 2262/0253; B32B 2555/02           (Cont.)

(86) International application number:
**PCT/US2021/018938**

(87) International publication number:
**WO 2021/168378 (26.08.2021 Gazette 2021/34)**

(54) **MULTI-LAYER ABSORBENT PRODUCT AND PROCESS FOR PREPARING ABSORBENT LAYER**

MEHRSCHICHTIGES ABSORBIERENDES PRODUKT UND VERFAHREN ZUM HERSTELLEN EINER ABSORBIERENDEN SCHICHT

PRODUIT ABSORBANT MULTI-COUCHE ET PROCÉDÉ DE PRÉPARATION D'UNE COUCHE ABSORBANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.02.2020 US 202062979261 P**

(43) Date of publication of application:
**28.12.2022 Bulletin 2022/52**

(73) Proprietor: **Polygreen Ltd.**
**22102 Nahariya (IL)**

(72) Inventors:
• **SHAHAL, Shai**
**Tel Aviv (IL)**
• **MEIRI, Zvi**
**Netanya (IL)**
• **ASH, Uri**
**1238700 Kahal (IL)**
• **TEPLITZKY, Peter**
**3307501 Haifa (IL)**

(74) Representative: **Wittop Koning, Tom Hugo**
**Biopatents IP Consultancy**
**Dagpauwooglaan 1**
**5691NW Son en Breugel (NL)**

(56) References cited:
WO-A1-2016/090330    WO-A1-2019/195271
WO-A1-2019/195272    US-A- 5 998 312
US-A1- 2002 193 516    US-A1- 2003 120 231
US-A1- 2004 054 341

(52) Cooperative Patent Classification (CPC): (Cont.)

C-Sets
**A61L 15/24, C08L 23/02;**
**A61L 15/60, C08L 35/06;**
**A61L 15/60, C08L 89/06**

**Description**

**Field of the Invention**

The present invention relates to a process for production of water-absorbing textile material such as non-woven, woven or other textile materials types or water-absorbing plastic yarn with textured surface and related absorbing products.

**Description of the Related Art**

In art there are known processes to manufacture an absorbent textile material, which can be divided in two distinct classes: a) processes with textile fibers and polymeric absorbents, b) processes with prefabricated textile materials and reactive liquid media.

Processes with prefabricated textile materials and reactive liquid media

Particularity of these processes consists in that, a traditional prefabricated textile material like nonwoven, woven, knitted or braided type, is impregnated with a fluid mass in solution, emulsion or suspension form, and a polymeric absorbent is generated in situ during the drying of the impregnated wet textile material, or after drying and applying of an adequate thermal treatment. Representative processes that are known in the art: b1) processes with polymerizable impregnation mass and b2) processes with cross-linkable impregnation mass.

Processes with polymerizable impregnation mass

In these processes, a liquid used for impregnation produces in-situ polymerization of partially neutralized acid vinyl monomers directly on a synthetic nonwoven substrate. These processes can be found in U.S. Patents 4,537,590; 4,540,454; 4,573,988; 4,676,784; 5,567,478; 5,962,068; 6,417,425 and 6,645,407 and patent applications US 2003/120231, WO 2019/195271 and WO 2016/090330.

Processes with cross-linkable impregnation mass

In these processes, the textile material prefabricated as: nonwoven, woven or other known type is impregnated with a fluid mass in the form of solution or emulsion, that contains polymers in dissolution or dispersion state, or in mixture with auxiliary materials which are cross-linking agents active at temperatures and induce generation in situ of a polymeric absorbent. In this class can be seen more variants:

i) Processes with impregnation mass that contain a self-crosslinking synthetic polymer.

Various processes are described in which a substantially linear acrylic polymer is cross linked through its pendant groups. The pendant groups will react with each other upon appropriate heating. Any combination of monomers that will undergo such reaction can be used as in U.S. Patents 4,057,521; 4,861,539; 4,962,172; 4,963,638; 5,280,079 and 5,413,747.
The impregnation mass based on self-crosslinking polymers has the disadvantage that the crosslinking is not complete and because of this reason more than 10 % of end product is lost during processes associated with extraction of monomers, initiators and organic solvents residue used during polymerization. Moreover, the free absorbency of the resulting cross-linked polymers has a low value (less than 50 g/g in 0.9 % NaCl solution), and the product is not safe.

ii) Processes with impregnation mass that contain synthetic polymers and cross-linking agents

It is further known from U.S. Patent 2,988,539; 3,393,168 and 3,514,419 that water swellable crosslinked carboxylic copolymers can be prepared. However, these prior art copolymers are all cross-linked during copolymerization or cross-linked after polymerization with subsequent neutralization of the carboxylic acid groups to form water swellable polyelectrolytes and hence these prior art polyelectrolytes cannot be cross-linked in situ as a coating on a substrate or as a flexible film thereof.
U.S. Patent 3,926,891; 3,980,663 and 4,155,957 present chemical structures of the principal classes of combinations that can be used as cross-linking agents for polymers with free carboxylic chemical functions. Also is shown that cross-linking reaction occurs based on mechanism known as nucleophilic displacement on saturated carbon. The carboxylate ion on polymer acts as nucleophile while cross-linking agent is the substrate for nucleophilic attack.

Cheng et al. in U.S. Patent 5,693,707 presents an aqueous polymer composition comprising 10 to 40 wt % of a polymer in water, the polymer consisting essentially of 20-90 wt % alpha, beta-ethylenically unsaturated carboxylic acid monomer, 10-80 wt % one or more softening monomers, the aqueous composition being adjusted to pH 4-6 with alkali metal hydroxide or alkaline earth metal hydroxide and further containing 0.1 to 3 wt % zirconium crosslinking salt. Such aqueous compositions are applied to nonwoven and woven substrates to make absorbent textile web.

Goldstein et al. in U.S. Patent 6,506,696 shows that the method of forming the high performance nonwoven webs of this invention comprises: applying an aqueous polymeric emulsion containing a polymer having dual cross-linkable functionality to a synthetic based nonwoven web, wherein the dual cross-linkable polymer incorporates acetoacetate functionality and carboxylic acid functionality; removing water and cross-linking the cross-linkable polymer with an effective amount of a polyaldehyde and an effective amount of a polyaziridine compound.

Soerens Dave Allen in U.S. Patent 7,205,259 presents an absorbent binder desiccant composition which is capable of spontaneous cross-linking after application to a substrate, at a temperature of about 120oC or less. The absorbent binder desiccant composition includes a monoethylenically unsaturated polymer, such as carboxylic acid, sulphonic acid, or phosphoric acid, or salts thereof, or a quaternary ammonium salt, and an acrylate or methacrylate ester that contains an alkoxysilane functionality, or a monomer capable of co-polymerization with a compound containing a trialkoxy silane functional group and subsequent reaction with water to form a silanol group, and a desiccant component. The absorbent binder desiccant composition is particularly suitable for use in manufacturing a wide variety of humidity control articles.

A diversity of chemical compositions (polymers and cross-linking agents) besides of multiple variants of control of cross-linking reactions are found in art in U.S. Patent 3,983,271; 4,066,584; 4,320,040; 4,418,163; 4,731,067; 4,855,179; 4,880,868; 4,888,238; 5,698,074; 5,997,791; 6,150,495; 6,162,541; 6,241,713; 6,773,746 and 6,824,650.

iii) Processes with impregnation mass that contain biopolymers and cross-linking agents

Weerawarna et al. in US Patent 7,300,965 provides a mixed polymer network having superabsorbent properties. The composition is obtainable by reacting a carboxyalkyl cellulose and a synthetic water-soluble polymer having carboxylic acid or carboxylic acid derivative substituents with a crosslinking agent. The cross-linking agent reacts with at least one of the carboxyalkyl cellulose or water-soluble polymer to provide the network. Suitable cross-linking agents include cross-linking agents that are reactive toward carboxylic acid groups.

Representative organic cross-linking agents that are reactive toward carboxylic acid groups include diols and polyols, diamines and polyamines, diepoxides and polyepoxides, polyoxazoline functionalized polymers, and aminols having one or more amino groups and one or more hydroxy groups.

Representative inorganic cross-linking agents that are reactive toward carboxylic acid groups include polyvalent cations and polycationic polymers. Exemplary inorganic cross-linking agents include aluminum chloride, aluminum sulfate, and ammonium zirconium carbonate with or without carboxylic acid ligands such as succinic acid (dicarboxylic acid), citric acid (tricarboxylic acid), and butane tetracarboxylic acid (tetracarboxylic acid). Water soluble salts of trivalent iron and divalent zinc and copper can be used as cross-linking agents. Representative carboxylic acid cross-linking agents includes di- and polycarboxylic acids. U.S. Patent 5,137,537; 5,183,707 and 5,190,563 describe the use of C2-C9 polycarboxylic acids that contain at least three carboxyl groups (e.g., citric acid and oxydisuccinic acid) as crosslinking agents. Suitable polycarboxylic acid crosslinking agents include citric acid, tartaric acid, malic acid, succinic acid, glutaric acid, citraconic acid, itaconic acid, tartrate monosuccinic acid, maleic acid, 1,2,3-propane tricarboxylic acid, 1,2,3,4-butanetetracarboxylic acid, all-cis-cyclopentane tetracarboxylic acid, tetrahydrofuran tetracarboxylic acid, 1,2,4,5-benzenetetracarboxylic acid, and benzenehexacarboxylic acid. The cross-linking can be achieved by heating at a temperature and for a period sufficient to effect the cross-linking. The carboxymethyl cellulose solution containing cross-linking agent or synthetic water-soluble polymer and cross-linking agent can be air-dried or solvent precipitated followed by cross-linking. Cross-linking time and temperature will depend on the cross-linking agent and polymers used.

Sun et al. in U.S.Patent 6,689,378 presents methods of immobilizing uncomplexed cyclodextrins and complexed cyclodextrins to polysaccharide containing substrates, such as cellulose fibers by covalently bonding. The cellulose/cyclodextrin compositions can be used in all types of cellulose fiber containing articles, such as tissues and personal care articles.

Useful polymeric anionic reactive compounds are compounds having repeating units containing two or more anionic functional groups that will covalently bond to hydroxyl groups of the substrate. Exemplary polymeric anionic reactive compounds include the ethylene/maleic anhydride copolymers described in U.S. Patent 4,210,489. Vinyl/maleic anhydride copolymers and copolymers of epichlorohydrin and maleic anhydride or phthalic anhydride are other examples. Copolymers of maleic anhydride with olefins can also be considered, including poly(styrene/maleic anhydride)

Copolymers and terpolymers of maleic anhydride that could be used are disclosed in U.S. Patent 4,242,408.

The cross-linkable impregnation mass, similar to polymerizable systems in situ have disadvantage that the cross-linking is not complete in conditions of thermal treatment mentioned, existing permanent the possibility that during uses of textile products to extract besides soluble polymer also cross-linking agent, this being more dangerous for the human healthy comparatively with the monomers or polymerization auxiliary residues. Presence of biopolymers in mixture for treating of textile materials, not contribute to improving the chemical transformation yield that occurs during cross-linking. Referring to uses of macromolecular cross-linking agents, these products being in their turn result of synthesis processes are not mentioned their purity or the extractability in aqueous solutions used as swelling media.

A particular drawback of known processes in the art for obtaining absorbent textiles is that those products are not ecological. Majority of polymeric absorbents discussed above, are products of synthesis, which because of their chemical structure have not the capacity of biodegradation in specific active biological media, i.e. the domestic compost. Moreover, absorbent textiles that contain biopolymers, although are biodegradable, have small values of free absorbency. Another drawback of known absorbent textiles is that the polymer network included in such textiles was obtained through the use of crosslinking agents that might present health hazards to end users.

Citation of any document herein is not intended as an admission that such document is pertinent prior art or considered material to the patentability of any claim of the present application. Any statement as to content or a date of any document is based on the information available to applicant at the time of filing and does not constitute an admission as to the correctness of such a statement.

## Summary of the invention

The present invention eliminates the disadvantages of processes known in art for obtaining absorbent textile products or absorbent textured plastic yarn products by providing a new process for treating a textile material or textured yarn as substrate and using the treated textile material or textured yarn with improved water absorbance and biodegradability as the basis to form a multi-layer absorbent product. The new process makes use of an aqueous textile-impregnating or textured yarn-impregnating polymeric composition in the form of a syrup (containing a high concentration, e.g., at least 20%-40%, of polymers in the solution) of at least two types of soluble polymers - a synthetic polymer and a natural polymer, with the syrupy solution being devoid of separate non-polymer crosslinking agent. The syrup of soluble polymers is deposited on the textile material or on the textured yarn in a uniformly spaced manner such that, when the textile material or the textured yarn with uniformly spaced deposits thereon is exposed to thermal/heat treatment, the polymers in the deposits impregnate the textile material or the textured surface of the plastic yarn and interpenetrate into the network of textile fibers of the textile material or the textured network of the plastic yarn and undergo self-crosslinking of the natural and synthetic polymer in the deposits.

The present invention further provides a multi-layer absorbent product for use in absorbent products such as diapers, feminine hygiene absorbent pads, panty liners, cleaning wipes, household or institutional cleaning or maintenance appliances, hand wipes, hand towels, personal, cosmetic or sanitary wipes, baby wipes, facial tissues, hygienic absorbent pads, panties, wound dressings and the like. This multi-layer absorbent product includes a first layer that is either liquid permeable or liquid impermeable (e.g., a bottom layer) and a layer of a treated nonwoven, woven or knitted textile material disposed next to and against the first layer. The layer of nonwoven, woven or knitted textile material has on top uniformly spaced deposits of a natural polymer crosslinked to a synthetic polymer in the absence of non-polymeric crosslinking agent. Each of the deposits of crosslinked natural and synthetic polymers is a continuous polymer network that covers the textile underneath the deposit and interpenetrates the network of textile fibers acting as a scaffold to interconnect the polymer network with the fiber network. The multi-layer absorbent product is also characterized by the high biodegradability of the deposits.

The present invention further provides a multi-layer absorbent product for use in the outdoor coverage or packing or packaging of vegetal agricultural crops such as fodder, hay or cotton, or crop residues such as straw, and their protection from rain, snow or ground moisture and the resultant damage from such uncontrolled humidity, to allow for their storage outdoors. This multi-layer absorbent product includes a first layer that is either liquid permeable or liquid impermeable (i.e., an upper layer) and a layer of a treated plastic yarn with textured surface, disposed next to, below and against the first layer. The layer of textured plastic yarn has on top uniformly spaced deposits of a natural polymer crosslinked to a synthetic polymer in the absence of non-polymeric crosslinking agent. Each of the deposits of crosslinked natural and synthetic polymers is a continuous polymer network that covers the textured yarn underneath the deposit and interpenetrates the network of textured plastic material acting as a scaffold to interconnect the polymer network with the plastic texture network. The multi-layer absorbent product is also characterized by the high biodegradability of the deposits.

The invention is defined by the appended claims.

## Brief description of the drawings

The invention will now be described with reference to the drawings, wherein:

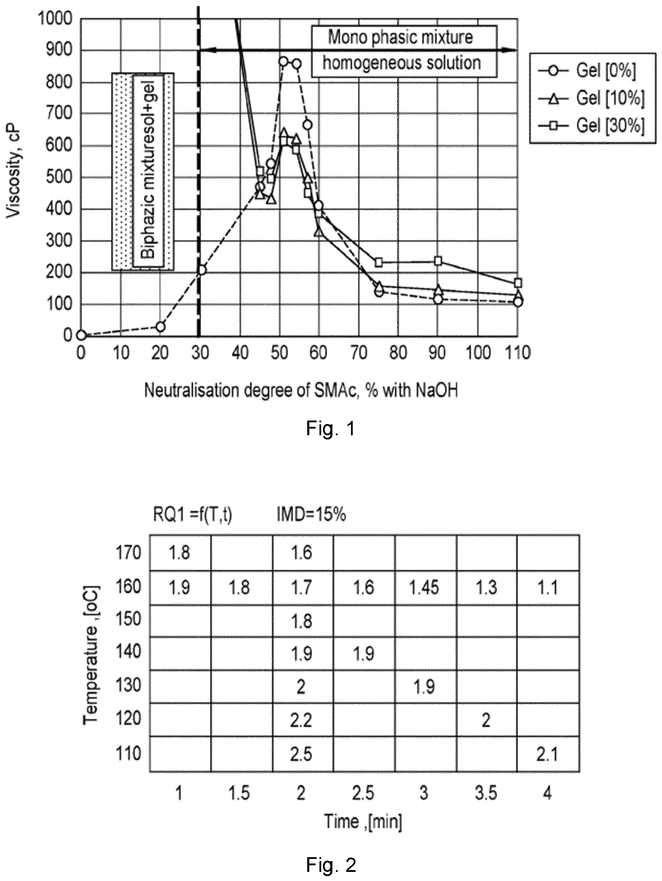

Figure 1 is a graph showing the correlation between the viscosity of SP-NP composite solutions, the degree of neutralization of the synthetic polymer (SP) and solution stability. The SP is represented by SMAC (Styrene Maleic Acid Copolymer) in salt form following neutralization with sodium hydroxide. The natural polymer (NP) is represented by gelatin. The following polymer solutions were tested: 100% SMAC; 90% SMAC and 10% Gelatin; 70% SMAC and 30 % Gelatin. The SMAC used had different degrees of neutralization as further explained in Example 1. From Figure 1 it is noted that the presence of the natural polymer Gelatin leads to a decrease in the viscosity of the polymer composite solution if the degree of neutralization of the synthetic polymer is between 30-60%. If the degree of neutralization is greater than 60%, the presence of the natural polymer causes an increase in the viscosity value of the composite solutions in comparison to same solution without natural polymer. With reference to the stability of the composite polymer solutions, it is noted that the composite solutions containing SMAC and gelatin are stable if the degree of neutralization of the synthetic polymer SMAC is greater than 30%.

Figure 2 is a graph showing the influence of thermal treatment to which is subdue a non-woven sample impregnated with the polymer composite solution containing synthetic polymer and gelatin on the relative absorbency RQ1.

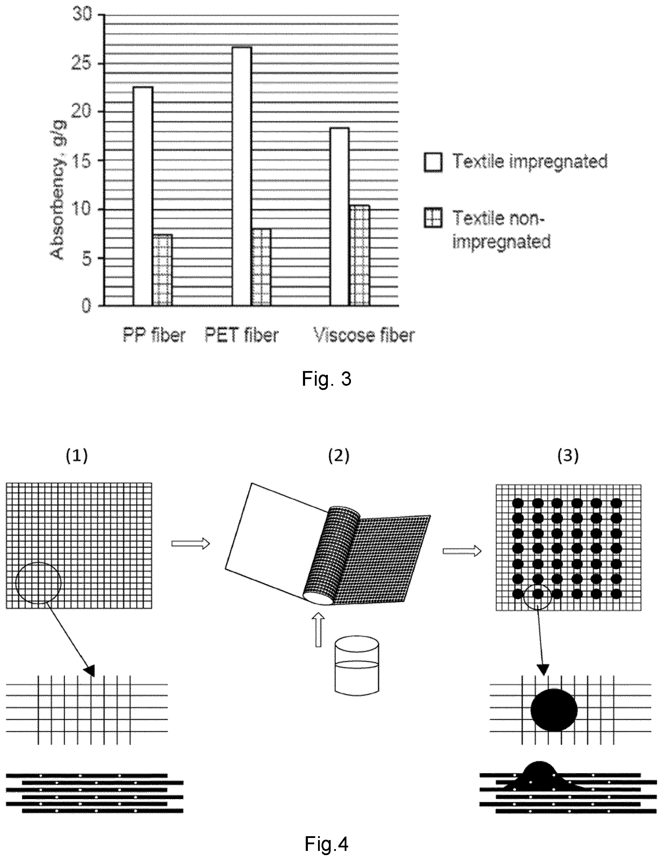

Figure 3 is a graph showing the influence of the type of fibers on water absorbency for textile impregnated with the polymer composite vs. a textile non-impregnated.

Figure 4 is a schematic illustration of the process that occurs between a sample of textile material or a textured plastic tarn and the polymer network deposited thereon under heating conditions to provide the textile composite material or the yarn composite where the polymer network interpenetrates the textile fiber network or the plastic surface pores. On the left, (1) presents a schematic illustration of a textile material, illustrating the fibers in small scale (top), large scale top-view (center) and large scale side view (bottom), where few layers of fibers can be seen, with dots representing in-plane fibers. In the middle (2), the process of roll printing is illustrated where the polymer solution is fed from a container into a perforated roll, from which it is deposited on the layer of textile material or plastic yarn; the dots on the layer represent the circular deposits. On the right (3), the finished composite including the substrate textile layer and dried, heat-treated polymer is illustrated: small scale (top) showing deposits larger than fibers and covering them, large scale top-view (center) showing one deposit on the fibers, and large scale side-view (bottom), illustrating the interpenetration of polymer in between the fibers to some depth, wrapping the fibers underneath the deposit, where this network of polymer and fiber or pore-walls composite act as a scaffold that holds the deposited polymer in place, on top of the substrate layer.

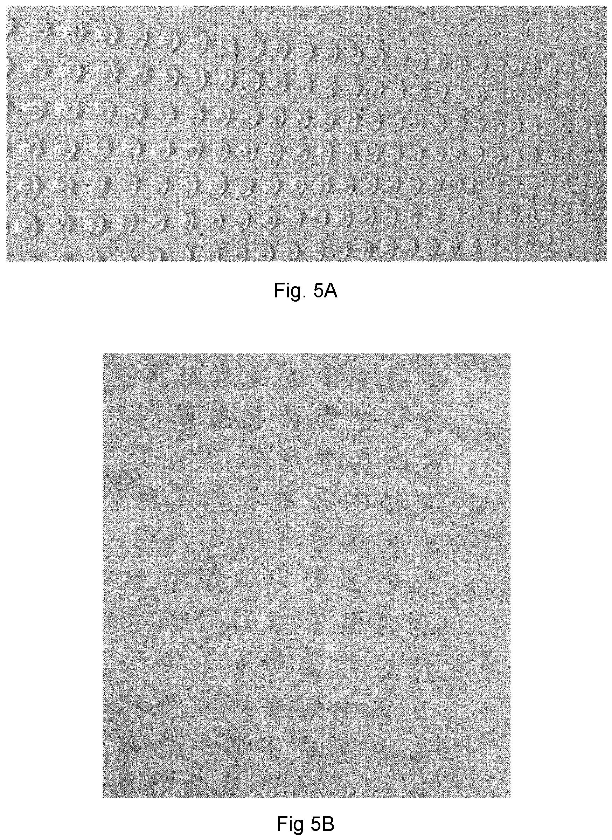

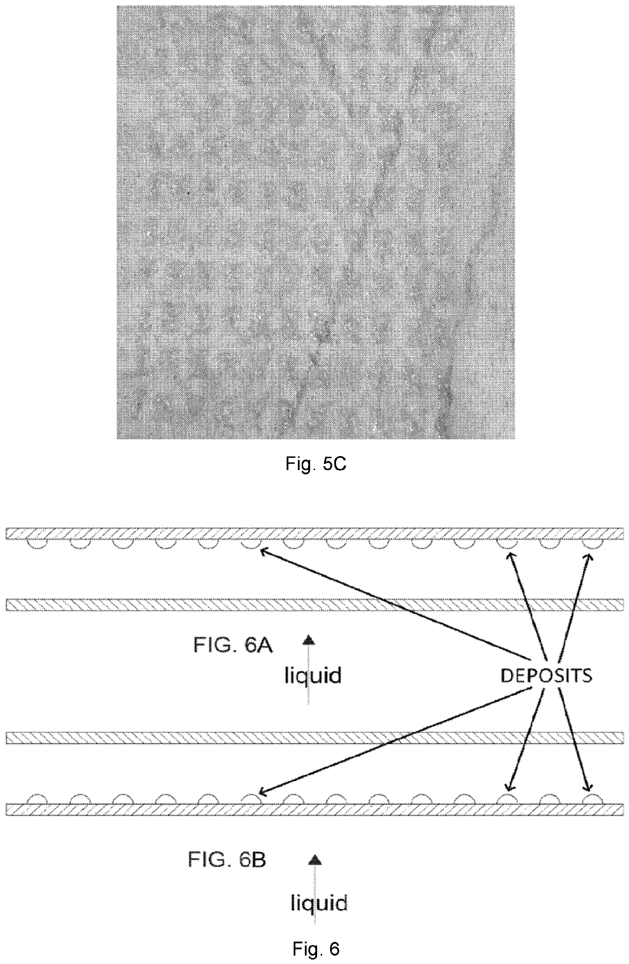

Figures 5A and 5B show uniformly spaced deposits of a concentrated solution of a natural polymer and a synthetic polymer on textile material, where before thermal treatment and self-crosslinking of the natural and synthetic polymers the deposits are half dome in shape (Fig. 5A) and become circular dots after thermal treatment (Fig. 5B, on a different textile material from Fig. 5A). Fig. 5C shows rectangular shaped deposits after thermal treatment.

Figures 6A and 6B are schematic illustrations showing a side view (not to scale) of two configurations of a two-layer absorbent product of the invention, where one of the two layers is a layer of nonwoven, woven or knitted textile material or textured plastic yarn with deposits of superabsorbent polymers (SAP) on one side and the other layer is a liquid permeable or liquid impermeable layer. The arrows represent the direction of liquid flow/penetration.

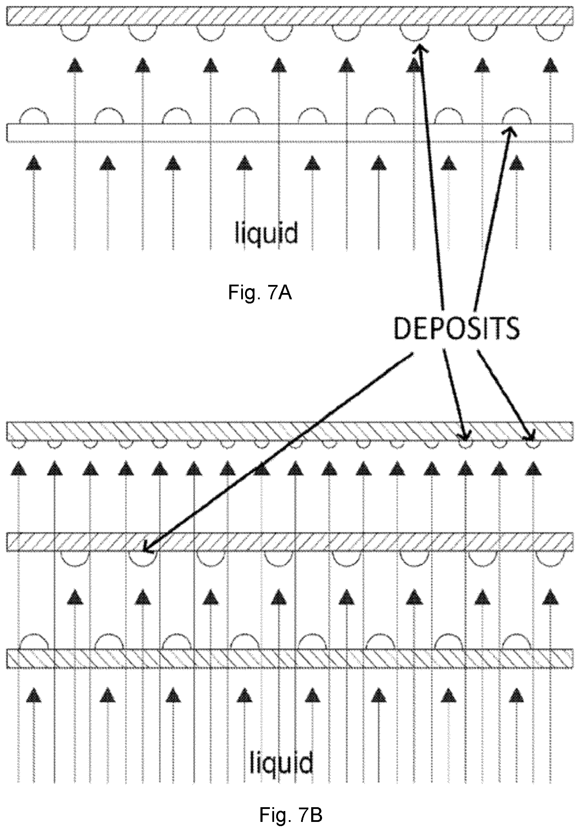

Figures 7A and 7B are schematic illustrations showing a side view (not to scale) of two different embodiments with two layers (Fig. 7A) or three layers (Fig. 7B) of nonwoven, woven or knitted textile material or textured plastic yarn with deposits of superabsorbent polymers (SAP) in the multi-layer absorbent product, which may have an additional layer(s) of a liquid permeable layer and/or a liquid impermeable layer (not shown) as an outer layer or layers of the product. The arrows represent the direction of liquid flow/penetration.

## Detailed description of the invention

The present invention provides a multi-layer absorbent product composed of at least a "first" layer that is either liquid permeable or liquid impermeable layer (such as the bottom sheet/layer in a diaper or feminine hygiene pads or liners) and a layer of textured plastic yarn or nonwoven, woven or knitted textile material composed of a network of fibers which is disposed next to and against the first liquid permeable or impermeable layer. A layer of nonwoven, woven or knitted textile material has an upper porous surface of fibers on which uniformly spaced deposits of a continuous polymer network of natural polymer crosslinked to a synthetic polymer (in the absence of non-polymeric crosslinking agent) cover the upper fibers of the textile material underneath each deposit and interpenetrates the network of fibers acting as a scaffold. A layer of textured plastic yarn also has an upper porous surface, but one created by non-penetrating holes, indentations, depressions, open bubbles, and other roughness-creating features formed by foaming, etching, cutting or other void-shaping methods. The depth of this surface porosity network should be sufficient to act as a scaffold for uniformly spaced deposits of a continuous polymer network of natural polymer crosslinked to a synthetic polymer (in the absence of non-polymeric crosslinking agent) that interpenetrate the surface pores underneath each deposit. This interpenetration of the network of fibers or plastic pores by the continuous polymer network interconnects the polymer network with the textile fiber network or with the surface pores in the plastic yarn, respectively. See, for instance, the schematic illustration in Fig. 4, where the continuous polymer network interpenetrates the network of textile fibers or network of pore-walls of the textured plastic yarn, interconnecting the two networks. Each of the uniformly spaced deposit covering the textile material directly underneath (impregnating and also interpenetrating the network of fibers underneath) is shown in Fig. 5B. Visually, the deposit appears to be "stuck" or "glued" to the textile material underneath. This provides a "glue-less" way to stick the deposit tightly to fiber network of the textile material substrate underneath, without any danger of the deposit falling apart or falling off because of vibration and speed (sheer forces) during production. The top surface of the layer of nonwoven, woven or knitted textile material with the uniformly spaced deposits is positioned next to and against the first liquid permeable or impermeable layer. The dry uniformly spaced deposits on one surface (e.g., top surface) of the layer of nonwoven, woven or knitted textile material confers to the impregnated textile superior free absorbance when exposed to aqueous fluid such as body fluids. When the impregnated textile with deposits thereon is exposed to aqueous liquids, it has sufficient free spaces that permit fast access and absorption of the liquid.

The density of the nonwoven, woven or knitted textile material (without the deposits), serving as substrate for the deposition/application of a thick and sticky syrup of natural and synthetic polymers (i.e., prior to being heat treated to self-crosslink the natural and synthetic polymers into a network of superabsorbent polymers (SAP)) is in a range between 20 to 150 g/m$^2$, preferably 20 to 60 g/m$^2$, more preferably 20 to 30 g/m$^2$. The textile material as substrate for the deposits of superabsorbent polymers (SAP) that make up the continuous polymer network include prefabricated textile materials such as nonwoven, woven or any other type known in art and commercially available, that are formed from synthetic fibers or natural fibers or a mixture of synthetic and natural fibers. The textile material may be pre-treated with additives or subject to surface treatment with chemicals that may confer on the fabric additional characteristics such as hydrophilicity, hydrophobicity or any other characteristics formed by processes known in the art of textile manufacture. Non-limiting examples of fibers in the textile material as carrier/substrate include synthetic fibers such as polypropylene (PP), polyester, polyvinyl alcohol, etc., and natural fibers such as polylactic acid (PLA), cellulose, viscose, bamboo, cotton, wool, paper, etc. The textile material may be biodegradable by itself, such as for example when made of PLA, or may become biodegradable when dry deposits of SAP are activated by contact with aqueous biological media, such as bodily fluids, to swell and transform into a gel-like material within the textile and on its surface. Preferably, the textile material as substrate is a nonwoven. A nonwoven PP textile material is a preferred embodiment of the substrate. Preferably, the textile material is thin so as to have the benefit of making the multi-layer absorbent product thinner than conventional, i.e., appears more two dimensional (2D) than the conventional fluffy media, which is three dimensional (3D).

The terms fabric, textile, web, with or without being combined with the word "material" are used in this specification interchangeably.

The thickness of the plastic yarn (without the deposits), serving as substrate for the deposition/application of a thick and sticky syrup of natural and synthetic polymers (i.e., prior to being heat treated to self-crosslink the natural and synthetic polymers into a network of superabsorbent polymers (SAP)) is in a range between 20 to 200 micrometers, preferably 20 to 80 micrometers. The density of the plastic yarn is in a range between 18 and 150 gr/m$^2$, preferably 18 to 77 gr/m$^2$. The plastic yarn as substrate for the deposits of superabsorbent polymers (SAP) that make up the continuous polymer network include prefabricated plastic yarn that may be produced by extrusion or any other method known in art and commercially available, made from synthetic polymers or natural polymers. Non-limiting examples of plastic materials that can form a carrier/substrate yarn include synthetic polymers such as polyethylene (PE), polypropylene (PP), polyester, polyvinyl alcohol, etc., and natural polymers such as polylactic acid (PLA) and cellulose. The plastic yarn may not be biodegradable, or may be biodegradable by itself, such as for example when made of cellulose, or may become biodegradable when dry deposits of SAP are activated by contact with water, to swell and transform into a gel-like material within the plastic yarn and on its surface. The plastic yarn may also be biodegradable following a prolonged weathering process such as exposure to light and air, or may be non-biodegradable following such treatment. Preferably, the plastic yarn as substrate is made of polyethylene (PE), which is a preferred embodiment of the substrate.

The weight of the deposits relative to the textile material or the plastic yarn as substrate/carrier is in a range of 100% to 600%, preferably 200% to 600%, more preferably 400% to 600%. This is the percent add-on weight to textile material substrate/carrier, where, for example, if a nonwoven textile material substrate/carrier is 22 grams per square meter (g/m$^2$ or gsm) with 88 gsm of the dry deposit (of SAP) deposited on the substrate/carrier, then the total weight is 110 gsm and the % add-on weight is 400%. Table 5 in Example 17 below shows an example of a textile material substrate/carrier where the add-on weight of the SAP deposited/printed on the nonwoven substrate is 175%. The textile material with the SAP deposits is still safe when in contact with the human body and is environmentally friendly, conferring a pronounced ecologic character to textile materials used.

The textile material with deposits of SAP as a superabsorbent layer in the multi-layer absorbent product has a water absorbency (free absorbance capability) in the range of at least 10% (e.g., at least 10%, 20%, 30%, 40%, 50%, 60%, 70%, 80%, 90%, or 100%) to 10 times higher than the absorbency of the same layer without the deposits (i.e., the non-impregnated textile material substrate), preferably at least 2 times higher than the absorbency of the same layer without said deposits, preferably at least 3 or 4 times, more preferably at least 5 or 6 times, most preferably at least 10 times.

On another measure of absorbency, the textile material or textured plastic yarn with deposits of SAP as a superabsorbent layer is capable of absorbing in the range of at least about 2 to 100 grams water per gram (g/g; gr/gr) on the basis of the layer, such as at least 3 gr/gr, at least 4 gr/gr, at least 5 gr/gr, at least 6 gr/gr, at least 7 gr/gr, at least 8 gr/gr, at least 9 gr/gr, at least 10 gr/gr, preferably at least 20, 30, 40, or 50 gr/gr, more preferably at least 60 gr/gr, 70 gr/gr, 80 gr/gr, or 90 gr/gr, most preferably at least 100 gr/gr.

The dry deposit of SAP on the textile material substrate or plastic yarn substrate is where 10% +/-1% of the SAP deposited on the substrate interpenetrate the network of fibers (e.g., tangle of fibers in a nonwoven), e.g., at least partially penetrating, within the textile material substrate or within the plastic yarn pores and the remainder 90% ± +/- 1% of the SAP is stuck to the upper porous surface of the textile substrate or the textured plastic yarn substrate to form a continuous SAP crosslinked polymer network that interconnects with the fiber network or network of pores (by sticking to and also interpenetrating the fiber network or the network of pores). Because SAP needs room to swell with water to take advantage of its superabsorbent properties and because, unlike pulp fibers that are loose and have space to swell with water, the fibers in nonwoven textile material used as substrate are tangled to each other in such a way that only allow for limited swelling ("blowing up") within the network of tangled fibers, the interpenetration of SAP into the fiber network is such that the space for swelling within the network of fibers is not limiting for the amount of SAP, e.g., about 10%, that interpenetrate the network of fibers. The SAP in the deposit that is stuck to the upper porous surface of the textile substrate or plastic substrate however is not constrained by space to swell.

The deposits on the textile material substrate as the layer of nonwoven, woven or knitted textile material are uniformly spaced with a spacing in a range of 1 to 7 mm, preferably 1 to 6 mm, more preferably about 2 mm.

Preferably, the uniformly spaced deposits on the textile material substrate are in the shape of circular dots or rectangles (Figs. 5B and 5C) but could be any shape or color that may be desirable, such as from a functional or aesthetic perspective. The size of the deposits is such that the space in between the deposits allows for fast penetration of liquids (such as that of water or body fluids, e.g., urine, menstrual fluid, etc.) for contacting the deposits on the first layer of textile material substrate or plastic yarn substrate in which the liquids comes into contact and then allowing some of the excess liquid not quickly absorbed by the first layer to move on to the deposits on any additional subsequent layers (in the direction of liquid flow or penetration) of textile material substrate or plastic yarn substrate in the multi-layer absorbent product.

In the deposits of SAP from crosslinked natural and synthetic polymers (crosslinked in the absence of non-polymeric crosslinking agents), the synthetic polymer has monomers bearing carboxylic acid or carboxylic acid anhydride groups and is preferably selected from linear or branched graft homo- or copolymers made from vinyl acidic monomers such as acrylic acid, maleic anhydride, itaconic anhydride and similar, optionally in association with other types of vinylic monomers that do not necessarily contain carboxylic acid functions. More preferably, the synthetic polymer is a copolymer based on maleic anhydride and/or maleic acid, preferably a copolymer of styrene maleic anhydride (SMA), copolymer of isobutylene and maleic anhydride (e.g., commercially available copolymers sold under the tradename ISOBAM™) or copolymer of methyl vinyl ether and maleic acid (e.g., commercially available copolymers sold under the tradename GANTREZ™).

Also in the deposits of SAP from crosslinked natural and synthetic polymers (crosslinked in the absence of non-polymeric crosslinking agents), natural polymer (NP) is a biopolymer selected from polysaccharides such as cellulose, alginate, dextran, chitosan, and the like; bio-polyesters and lignin (in native forms or modified by chemical or enzymatic hydrolysis); guar; starch; water soluble phospholipids such as lecithin; polypeptides or proteins such as gelatin, albumin, soybean protein, collagen, collagenic biopolymer, collagen hydrolysate, and casein. The natural polymer has amino and/or hydroxyl groups capable of cross-linking to COOH groups in said synthetic polymer under high temperature conditions and for selected periods of time. The natural polymer is a biopolymer that functions as both cross-linking agent of the synthetic polymer and as activator of biodegradation processes. Thus, the natural polymer activates the biodegradation of the synthetic polymer and confers to the system superior biodegradability.

The natural polymer (NP) is preferably a commercially available biopolymer which belong to the following classes of substances: proteins, carbohydrates, bio-polyesters or lignin (as native forms or modified by chemical or enzymatic hydrolysis); preferably the proteins and carbohydrates are soluble in water and have the following characteristics:

a) The average molecular weight from 5 to 100 kDa, preferably from 25 to 50 kDa, more preferably from 75 to 100 kDa
b) Free chemical functions as $NH_2$; $OH-CH_2$; $OH-C_6H_{3-5}$; as single type or more types which have content from 0.001 to 0.01 mol /grams.

The multi-layer absorbent product based on textile material substrate layers according to the invention is for use in absorbent products such as diapers, feminine hygiene absorbent pads, panty liners, cleaning wipes, household or institutional cleaning or maintenance appliances, hand wipes, hand towels, personal, cosmetic or sanitary wipes, baby wipes, facial tissues, hygienic absorbent pads, panties, wound dressings and the like. Where more absorbent layers are needed, such as in diapers, more than one layer of the textile material with uniformly spaced deposits of SAP thereon may be used in the multi-layer absorbent product. For instance, where desirable, there may be 2-5 layers in the multi-layer absorbent product.

The multi-layer absorbent product based on textured plastic yarn layers according to the invention is for use in in the outdoor coverage or packing or packaging of vegetal agricultural crops such as fodder, hay or cotton, or crop residues such as straw, and their protection from rain, snow or ground moisture and the resultant damage from such uncontrolled humidity, to allow for their storage outdoors. Where more absorbent layers are needed, such as to mitigate heavy rainfall, more than one layer of the textured plastic yarn with uniformly spaced deposits of SAP thereon may be used in the multi-layer absorbent product. For instance, where desirable, there may be 2-5 layers in the multi-layer absorbent product.

When only two layers are present in the multi-layer absorbent product (Figs.6A and 6B), and one layer is a layer of the textured plastic yarn with uniformly spaced deposits of SAP then the other layer is either a liquid permeable layer or a liquid impermeable layer. The liquid permeable layer or liquid impermeable layer can be any liquid permeable or liquid impermeable layer that is known and used in the art of either textile materials, woven, non-woven or knitted, or another plastic yarn that can be textured or smooth. If another plastic yarn is used as the second layer, the liquid permeability can be achieved by perforating the plastic yarn with small, penetrating holes. The size, shape and density of the holes can vary to adjust the liquid permeability or impermeability, for example, to allow for water vapor penetration through the second, upper layer without SAP deposits, while blocking or minimizing liquid water penetration through this layer. In a similar manner, the first layer with uniformly spaced SAP deposits can also be partially water-permeable, for example, by perforating it with small penetrating holes, that will allow water vapor penetration but will block or minimize liquid water permeability.

When only two layers are present in the multi-layer absorbent product (Figs.6A and 6B), and one layer is a layer of the textile material with uniformly spaced deposits of SAP, then the other layer is either a liquid permeable layer or a liquid impermeable layer. The liquid permeable layer or liquid impermeable layer can be any liquid permeable or liquid impermeable layer that is known and used in the art of absorbent products such as diapers, feminine hygiene absorbent pads, panty liners, cleaning wipes, household or institutional cleaning or maintenance appliances, hand wipes, hand towels, personal, cosmetic or sanitary wipes, baby wipes, facial tissues, hygienic absorbent pads, panties, wound dressings and the like. In the case of a two-layer absorbent product, the liquid permeable layer may be another layer (a second layer) of the textile material with uniformly spaced deposits of SAP. Preferably, the two layers of textile material with uniformly spaced deposits of SAP thereon are arranged where the top surface of the second layer is placed against the top surface of the first layer such that the uniformly spaced deposits on the top surfaces of the first and second layers do not overlap and contact each other (Fig. 7A). Thus, this arrangement of the two layers of textile material with uniformly spaced deposits of SAP provides a complementary design that avoids a gel blocking effect, where the swelling of the SAP deposits from absorption of liquid into a gel would undesirably close off (block) areas of the layer, such as the spaces between deposits, thereby allowing liquid to pass through to the next layer of uniformly spaced deposits for further absorption.

In addition to the two layers of the multi-layer absorbent product based on textile material of the present invention as described above, there may be one or more additional liquid permeable layers. At least one of these additional liquid permeable layers can be a further layer of nonwoven, woven or knitted textile material having a top surface with uniformly spaced deposits of SAP. The more layers of the textile material with uniformly spaced deposits of SAP in the multi-layer absorbent product, the more the total amount of SAP is available for absorbing liquids. For small amounts of liquid/fluids, such as in feminine care products for light menstrual flow, one layer with SAP deposits in the multi-layer absorbent product may be sufficient. However, where there is more fluid discharge that needs to be absorbed, such as in diapers, there can be three or even four layers with SAP deposits to provide a higher total amount of SAP available for greater liquid absorbancy. Having the deposits of SAP on different layers can help avoid a gel blocking effect by limiting the add-on weight of the deposits of SAP on a single layer of textile material. Another feature in optimizing high total amount of SAP deposits, total absorbance and absorbance speed for the multi-layer absorbent product of the invention is to have more layers that have SAP deposits but with different deposit sizes in the different layers and with relative positioning of the deposits in the different layers to avoid gel blocking effects. See, for example, Fig. 7B, where different size deposits on different layers are positioned relative to each other. By avoiding gel blocking effects, the maximum amount of SAP in the layers of the multi-absorbent product of the invention can absorb the maximum amount of liquid in the minimum amount of time.

The superabsorbent layer of nonwoven, woven or knitted textile material with uniformly spaced deposits of SAP is lightweight and thin and can be used in a feminine hygiene product marketed for the first and last day of the monthly menstrual period to provide a thin airable soft and skin friendly solution for what desirable and advantageous in such thin feminine hygiene products, with the added benefit that the presence of the natural polymer crosslinked to synthetic polymer in the SAP provides biodegradability.

Conventionally, the process for producing absorbent products, such as diapers and feminine hygiene pads, involves randomly distributing and mixing superabsorbent polymer in granulated powder form inside a fluffy pulp media to create a fluffy think pulp layer containing the granulated SAP as a thick absorbent core. This absorbent core is then wrapped in nonwoven or other wrapping layers to further create a thick wrapped absorbent core, where the wrapped absorbent core is cut into certain sizes. The sized absorbent core is integrated into additional layers of nonwoven/other material wrapping. In the conventional process, the crosslinking to form the SAP is done "offline" and then mixed with the fluffy pulp media. By contrast, the present invention provides a process where the polymer syrup solution is first applied to the textile material and then crosslinked (self-crosslinked) "onboard" the textile material as opposed to "offline".

The present invention thus provides a process for producing the superabsorbent layer based on textile material that can be integrated into a multi-layer absorbent product such as diapers and feminine hygiene pads with added benefits, including:

Benefits for the hygiene manufacturers, such as:

Thinner hygiene products - no fluff is needed;
Lighter hygiene products - simpler design of the product and less "packaging";
More "airable" - breathable Hygiene products;
Potentially eliminating absorbent core manufacturing - no core absorbent production bottle neck and hence faster production;
Eliminating work environment hazards such as (1) SAP dust (2) SAP particles falling from the vibrating machines and causing slippery floor environment (both are known as major concerns for manufacturers) - SAP deposits stuck on the textile material substrate according the present invention will not fall apart or fall off the textile material; and
Option to offer a more biodegradable solution.

Benefits for the nonwoven manufacturers, such as:

New product functionality for nonwoven manufacturer - from "smart packaging for SAP" to "functional packaging" - packaging that also take part in the core functionality of the hygiene product - to absorb liquid
Allows the nonwoven industry to take a larger share in the hygiene value chain by providing additional functionality

The process for preparing the layer of nonwoven, woven or knitted textile material or textured plastic yarn with uniformly spaced deposits of a natural polymer crosslinked to a synthetic polymer used in the multi-layer absorbent product according to the present invention involves depositing on a nonwoven, woven or knitted textile material or on a textured plastic yarn (as substrate having a network of fibers or pores and pore walls) uniformly spaced deposits of a polymeric composite aqueous solution in the form of a thick and sticky syrup for use in improving water absorbency of the textile material or creating water absorbency of the plastic yarn. The polymeric composite solution includes a natural polymer and a synthetic polymer having monomers bearing carboxylic acid or carboxylic acid anhydride groups, where the natural and synthetic polymers in the composite solution are capable of undergoing self-crosslinking under controlled conditions of temperature and time and in the absence of non-polymeric crosslinking agent. The concentration of the combination of natural and synthetic polymers is in a range of 20-40% w/w of the polymeric composite solution. The textile material or textured plastic yarn having uniformly spaced deposits thereon are dried and then thermally treated (heat curing) under controlled conditions of temperature and time so that the natural and synthetic polymers in the deposit undergo self-crosslinking in the absence of non-polymeric crosslinking agent in the deposits to form SAP as a continuous polymer network that covers the fibers or pores and pore walls underneath the deposit and interpenetrates the network of fibers or pore walls acting as a scaffold to interconnect the SAP polymer network with the fiber network or network of plastic pore walls.

In the depositing step, the uniformly spaced deposits are deposited ("printed") on a continuous roll of nonwoven, woven or knitted textile material or on textured plastic yarn preferably by rotary screen printing with a perforated cylinder in a 3D dot printing device. The 3D printing of polymer syrup as uniformly spaced deposits on a continuous roll of the textile material substrate or of the textured plastic yarn is preferably done with equipment in which rotary screen printing with a perforated cylinder is integrated with downstream continuous processing such as a downstream oven for drying and a heat curing thermal treatment of the deposits.

After the thermal treating step to self-crosslink the natural and synthetic polymers in the deposits, individual sheets of textured plastic yarn or of nonwoven, woven or knitted textile material with the uniformly spaced deposits can be cut from the continuous roll and used as a superabsorbent layer for assembly with other layers into the multi-layer absorbent product according to the invention. Alternatively, the multi-layer absorbent product may be assembled from continuous rolls of the different layer materials and then cut. The cutting and/or assembly may be done at a different facility from the 3D printing and thermal treatment.

The textured plastic yarn or nonwoven, woven or knitted textile material used in the process for the depositing step (i.e., prior to depositing) has a density between 20 to 150 g/m$^2$, preferably 20 to 60 g/m$^2$, more preferably 20 to 30 g/m$^2$.

After being thermally treated in the present process, the weight of the deposits relative to the textile material or to the plastic yarn is in a range of 100% to 600%, preferably 200% to 600%, more preferably 400% to 600%.

The deposits printed on the textile material substrate are uniformly spaced, with the spacing in a range of 1 to 7 mm, preferably 1 to 6 mm, more preferably about 2 mm and preferably printed as half domes, which become circular dots on the surface of the textile substrate when dried and thermally treated (Fig. 5B), or printed in a rectangular shape. It would be appreciated by those in the art that the printing of the deposits could be of any shape (or color by introducing a coloring agent) that may be desirable, such as from a functional or aesthetic perspective. One only needs to create a cylinder for rotary screen printing that has the desired shape or shapes (may be multiple shapes present).

The synthetic polymer (SP) used for self-crosslinking to the natural polymer in the deposit has monomers bearing carboxylic acid or carboxylic acid anhydride groups and is preferably selected from linear or branched graft homo- or copolymers made from vinyl acidic monomers such as acrylic acid, maleic anhydride, itaconic anhydride and similar, optionally in association with other types of vinylic monomers that do not necessarily contain carboxylic acid functions.

Other preferred embodiments of the synthetic polymer are a copolymer based on maleic anhydride and/or maleic acid, preferably a copolymer of styrene maleic anhydride (SMA), copolymer of isobutylene and maleic anhydride (e.g., commercially available copolymers sold under the tradename ISOBAM™) or copolymer of methyl vinyl ether and maleic acid (e.g., commercially available copolymers sold under the tradename GANTREZ™).

Preferably, the synthetic polymer is poly(decyl vinyl ether-alt-maleic anhydride), poly(ethyl vinyl ether-alt-maleic anhydride), poly(maleic acid-co-propene), poly(n-butyl vinyl ether-alt-maleic anhydride), poly(octadecene-alt-maleic anhydride), poly(propylene-alt-maleic acid) or poly(maleic acid-co-dodecyl methacrylate).

The synthetic polymer (SP) may be a commercially available polymer, having the following characteristics:

a) linear copolymers or branched graft homo- or copolymers that contain vinyl acidic monomers as: acrylic acid, maleic anhydride, itaconic anhydride and similar in association or not with other types of vinylic monomers which does not contain carboxylic groups.
b) content of total free carboxylic groups from 0.009-0.0095 mol /gram to 0.01-0.015 mol/grams
c) Free carboxylic groups of synthetic polymer, SP are in salt state, corresponding to a degree of neutralization between 49 - 99 %, preferable between 60 - 95%, and most preferable between 65 - 90%;
d) The salt state of synthetic polymer is obtained by using strong inorganic base substances such as hydroxides, bicarbonates or carbonates of lithium, sodium, potassium or ammonium, preferred hydroxides of lithium, sodium, potassium or ammonium.
e) The average molecular weight of synthetic polymer, SP is from 50 kDa to 1,000 kDa, preferably from 100 kDa to 750 kDa, and most preferably from 150 kDa to 500 kDa.

The natural polymer (NP) used for self-crosslinking to the synthetic polymer in the deposit is a biopolymer selected from polysaccharides such as cellulose, alginate, dextran, chitosan, and the like; polyesters and lignin (in native forms or modified by chemical or enzymatic hydrolysis); guar; starch; water soluble phospholipids such as lecithin; polypeptides or proteins such as gelatin, albumin, soybean protein, collagen, collagenic biopolymer, collagen hydrolysate, and casein. The natural polymer has amino and/or hydroxyl groups capable of cross-linking to COOH groups in said synthetic polymer under high temperature conditions and for selected periods of time.

The natural polymer NP is preferably water-soluble phospholipids such as lecithin; polypeptides or proteins such as gelatin, albumin and the like; or polysaccharides such as cellulose, alginate, dextran, chitosan, and the like, that have at least one of the following characteristics:

a) average molecular weight from 5 to 100 kDa, preferably from 25 to 50 kDa, more preferably from 75 to 100 kDa.
b) free chemical groups such as amine or hydroxyl in a relative concentration from 0.001 to 0.01 mole/grams NP.
c) hydrophilic, capable of forming hydrogels in aqueous environment and capable of integration within the textile fibers.

According to preferred embodiments, the biopolymer used in the invention has the capability of cross-linking due to its NH$_2$ or OH groups that crosslink to COOH groups in the synthetic polymer under high temperature conditions and for selected periods of time, to form ester and amide bonds between polymers skeletons. Furthermore, the biopolymer activates the biodegradation of the textile impregnated with composite polymer solution of SP and NP.

The polymeric composite aqueous solution used in the process according to the present invention for preparing the layer of nonwoven, woven or knitted textile material or the layer of textured plastic yarn with uniformly spaced deposits of a natural polymer crosslinked to a synthetic polymer which is part of the multi-layer absorbent product can be prepared as follows:

a) preparation of alkaline base solution
b) preparation of aqueous solution of synthetic polymer (SP) in acidic form, which is treated afterwards with the alkaline solution from (a) to obtain the salt form, at a concentration of between 1% and 10%, preferably between 2 and 5% by weight,
c) preparation under heating of aqueous solution of natural polymer (NP) at a concentration between 1% and 10%, preferably between 2 and 5% by weight
d) mixing under heating and stirring the SP solution obtained in (b) in salt form, with the NP solution obtained in (c) to obtain aqueous stable composite solution of polymers suitable to be used as impregnation mass that confers high water absorbance to textile materials
and optionally
e) adding to the aqueous composite solution obtained in (d) at least one auxiliary material selected from the list of plasticizers, surface agents, deodorants, perfume and preservatives.

The polymers composite solution used is a stable solution of polymer materials SP and NP, without phase separation under conditions of storage or ulterior processing and may further contain at least one compatible additive used in the textile industry such as plasticizers, dyes, antibacterial agents, surfactants, deodorants, perfume, preservatives, etc., in quantities that are correlated with other properties than absorbency and without affecting the water absorptive performance or biodegradability of the textile material. The polymers composite solution is suitable for impregnation into fabrics and also suitable for self-crosslinking under controlled conditions of temperature and time.

Without being bound to theory, the polymer composite solution can interpenetrate within the textile fibers or the textured plastic yarn pore walls and self-crosslink under conditions of thermal treatment at temperatures between 100 to 250°C and during periods of preferably between 2 to 30 minutes.

Impregnation of the textile material is done using any equipment known in art, for example spray devices; foulard; roll coating; reverse roll coating; knife devices etc., preferably using rotary screen printing with a perforated cylinder in a 3D dot printer device.

## EXAMPLES

### Test methods

1. Characterization of polymer solutions containing synthetic polymer, natural polymer and auxiliary

- The viscosity of the solution was evaluated using a viscosimeter ViscoStar Plus, Fungilab, Spain using a volume of solution correlated to the type of spindle L1 at 25°C temperature.
- The stability of the polymer solution used for impregnation was assessed to be stable or unstable if the analyte solution showed sediment after centrifugation of a volume of 25 ml solution at 5000 rpm for 30 minutes. Tests were performed with a laboratory centrifuge GLC-2B Sorvall, Thermo Scientific at ambient temperature.

2. Free absorbency

The following measurements are made:

Mtex ,[grams] mass of the dried textile material used for impregnation by weighing at a semianalytical balance
Mtwstart ,[grams]- mass of wet starting material by weighing the sample
Mtid -[grams]- mass of drained textile material after impregnation
MIC-[grams]- mass of dried polymeric composite material found in the textile after impregnation evaluated by the formula :
MIC= Mtex*IMD/100, grams

The degree of impregnation of a textile material is established using the formula:

$$[IMD]dry= MIC/( Mtex+ MIC )*100, \%$$

2.1 Absorbency evaluation of textile materials:

Absorbency of textile material non-impregnated Q1 consists of introducing the textile sample, M-tex into a 150 ml volume of liquid to which the absorbance value is desired so that the entire surface of the textile material is covered by the liquid and the contact is maintained without shaking for 60 minutes. Ulterior, the material is removed from the liquid, it hangs in a vertical position to drain excess liquid for 15 minutes. The drained textile material sample is weighed and the value obtained is recorded as M-tex-wet.

Absorbency of non-impregnated textile material Q1 is obtained by using the formula:

$$Q1 = (M\text{-tex wet}-M\text{-tex})/M\text{-tex} , (g/g)$$

2.2 Absorbency of impregnated textile material Q2

The textile sample is weighted at technical balance obtaining the value Mtex. Then is impregnated with a chosen mass of liquid:distilled water, an impregnating solution or other type of solution by using a laboratory spray device . The wet material thus obtained is weighted again and the value is Mtwstart . If this value is higher than the impregnation degree (IMD) - pre-established the wet sample is subdue to a squeezing process with the aid of a glass rod to eliminate the excess of impregnation liquid so that it finally is obtained a wet sample with the mass Mtid . Next, the wet sample is kept for 30 minutes, in a closed glass beaker to avoid evaporation of the liquid.

The absorbency of textile material sample is obtained with formula:

$$Q2\text{-}TEXIC = [Mtid – (Mtex+M \text{ IC })]/(Mtex+M \text{ IC}), (g/g)$$

2.3. Relative absorbency

RQ represents the ratio between absorbency of an impregnated textile material Q2 and absorbency of the same textile material non-impregnated Q1.

Relative absorbency is calculated by using the formula:

$$[RQ]1 = Q2/Q1$$

or

$$[RQ]2 = [(RQ1)\text{-}1]*100, (\%)$$

Having now generally described the invention, the same will be more readily understood through reference to the following example which is provided by way of illustration and is not intended to be limiting of the present invention.

**Example 1**

Stock solutions of synthetic polymer, natural polymer and inorganic base are prepared as follows:

a) Stock of Synthetic Polymer Solution:
42.6 g of SMAc styrene / maleic acid copolymer [prepared as in U.S.Patent 7,985,819] in powder form having an 8% moisture content with an average molecular weight of 450,000 Da containing 0.0091 mol/g free carboxylic groups together with 358 g of demineralized water were added to a mixing vessel with agitation and the content was mixed for 1 hour at 80°C to complete dissolution of the synthetic polymer and is end by cooling the polymer solution to 40°C. Finally, 400 g of SMAc polymer solution of 10% by dry weight is obtained.
b) Stock of sodium hydroxide solution
Is prepared 400 g of NaOH solution of 10% dry weight (from 98.9% pure sodium hydroxide pellets) and demineralized water using a mixing vessel with agitation fitted with a heating-cooling jacket.
c) Stock of natural polymer solution
400 g of gelatin type A solution with 200 Bloom and 14% moisture content (as natural polymer-NP) of 10% dry weight were prepared by dissolving 46.5 g of natural polymer in 354 g of demineralized water using a mixing vessel by stirring with a rotor speed not higher than 60 rpm, during a period of 1 hour at a temperature of 40°C so as to ensure the perfect homogenization of the solution.

Further, the 3 types of solutions prepared above were used to prepare 3 sets of composite solutions by diluting with demineralized water the stock solutions as follows:

Set-SOL1 without gelatin made up of 12 solutions of 3% concentration in which the synthetic polymer has a different degree of neutralization between 0% and 110%.
Set-SOL-2 containing gelatin in proportion of 10% to SMAc consisting of 12 solutions of 3% concentration in which the synthetic polymer has a different degree of neutralization between 0% and 110% and Set-SOL-3 which contains gelatin in a proportion of 30% to SMAc consisting of 12 solutions of 3% concentration in which the synthetic polymer has a different degree of neutralization ranging between 0% and 110%.

All composite solutions corresponding to the three sets were characterized in terms of their viscosity and stability in the sense that the sample is unstable if it contains the insoluble phase and that the sample is stable if it is a homogeneous solution without the insoluble phase. The results obtained are presented in Table 1 and Figure 1.
From Figure 1 it is noted that the presence of the natural polymer leads to a decrease in the viscosity of the synthetic polymer if the degree of neutralization of the synthetic polymer is between 30-60%. If the degree of neutralization is greater than 60%, the presence of the biopolymer determines the increase in the viscosity value of the composite solutions. From the point of view of the stability of the composite solutions, this is dependent on the degree of neutralization of the synthetic polymer. It has been noted that composite systems containing SMAc and gelatin are stable if the degree of neutralization of the synthetic polymer is greater than 30%.

Table 1 Influence of the degree of neutralization of the synthetic polymer SPS and of the natural polymer NP content (as gelatin type A with 200 Bloom) in the composition SP: NP) on solutions' viscosity [h, (cP)] of 3% by dry weight.

| Neutralization degree of SP, [%] | Gel [0%] $\eta$; [cP] | Gel [10%] $\eta$; [cP] | Gel [30%] $\eta$; [cP] |
|---|---|---|---|
| 0 | 4.3 | 2000 | 2000 |
| 2 | 28.7 | 1900 | 1900 |
| 3 | 209.6 | 1800 | 1800 |
| 45 | 473.6 | 454.3 | 519.2 |
| 48 | 543.7 | 432.8 | 491.3 |
| 51 | 867.5 | 634.1 | 612.6 |
| 54 | 858 | 624.8 | 589.2 |
| 57 | 667.1 | 496.7 | 452.2 |
| 60 | 409.5 | 330.6 | 386.8 |
| 75 | 141.7 | 160 | 231.4 |
| 90 | 120.1 | 147.3 | 234.8 |
| 110 | 110.4 | 129.5 | 165.9 |

**Examples 2 to 5**

In these examples, is presented the absorbency value to demineralized water (conductivity 2 micro S) of some nonwoven fabrics containing viscose fiber with density of 50 g/m$^2$ with different impregnation degree with polymeric solutions' concentration of 1% by weight containing SMAc (mentioned in Example 1) with neutralization degree of 59% generated with NaOH and various values of gelatin content and which additionally contains 1.5% by dry weight to the content of the polymeric composition.
The experiments were conducted as follows:
Textile materials having a mass of 0.5g were impregnated with composite solutions prepared according to the method described in Example 1 by spraying with a laboratory device so as finally to obtain the impregnation degree pre-established, IMD, to the dry mass of the sample of dried fabric material. Further, the wet textile samples were placed in a hanging state in an oven with air circulation, preheated to 180°C and are maintained at this temperature for 4 minutes.

Finally, the sample was removed from the oven and allowed to cool at ambient temperature. Then the textile material was subjected to the absorbency test according to the methodology described in the chapter "Test methods".

Experimental conditions and results are shown in Table 2.

Table 2 The influence of gelatin content in the polymeric solution for impregnation and the impregnation degree on the absorbency of impregnated nonwoven

| Example cod | $[Q\text{-tex}]_{water}$ | Gelatin in Composite, % | IMD, % | $Q_2$-texic, (g/g) | $RQ_1$ |
|---|---|---|---|---|---|
| Exp-2 | 9.19 | 7.6 | 8 | 15.79 | 1.71 |
| Exp-3 | 9.19 | 9.7 | 10 | 12.08 | 1.39 |
| Exp-4 | 9.19 | 13.5 | 8 | 14.07 | 1.53 |
| Exp-5 | 9.19 | 17.6 | 12 | 15.49 | 1.68 |

## Examples 6-12

In these examples are presented the impregnation of a textile material consisting of viscose fibers with a density of 50 $g/m^2$ using a solution of synthetic polymer SMAc with neutralization degree of 64% done by using NaOH, 3.6% gelatin to the polymeric composite at degree of impregnation of 20% with thermal treatment of 200°C for 100 seconds using the same oven with air circulation mentioned in the previous examples. Finally, the absorbency of the textile sample was evaluated against various liquid media represented by simulated fluid secreted by the human body (Margareth R.C. Marques et al, Simulated Biological Fluids with Possible Application in Dissolution Testing, Dissolution Technologies AUGUST 2011, 1)

The results are presented in Table 3 below.

Table3 Influence of the liquid medium's composition on the absorbency of textile materials impregnated with polymeric composites containing synthetic polymer and gelatin

| Examples code | Liquid media | M-tex [g] | IMD dry % | $Q_1$ [g/g] | Q2 tex [g/g] |
|---|---|---|---|---|---|
| Exp-6 | Distillated water 0.0002mS | 0.5 | 15 | 29.03 | 50.48 |
| Exp-7 | Tap water 4 mS | 0.5 | 15 | 16.20 | 28.17 |
| Exp-8 | Salt water 0.9% 15.4mS | 0.5 | 15 | 5.89 | 10.24 |
| Exp-9 | Bovine Milk (3%) | 0.5 | 15 | 5.11 | 8.88 |
| Exp-10 | Simulated Human Sweat [1] | 0.5 | 15 | 2.81 | 4.89 |
| Exp-11 | Simulated Wound Fluids[1] | 0.5 | 15 | 6.28 | 10.92 |
| Exp-12 | Simulated Saliva[1] | 0.5 | 15 | 7.63 | 13.26 |

## Example 13

In this example is presented the influence of the temperature and the time of the thermal treatment at what is subjected a sample of textile material with a density of 50 $g/m^2$ in order to obtain an absorbent textile material on the relative absorption RQ1. The results are presented in Figure 2.

The data obtained show that the thermal treatment of the impregnated fabrics must be in such manner as to obtain the highest value for absorbency. The lower values of the maximum result either because of a low crosslinking degree or because the degree of crosslinking of the polymeric composite is too high.

## Example 14

This example shows the influence of the type of fiber from which the fabric is done subjected to impregnation.

For this purpose, a composite polymer solution having the chemical composition coinciding with Exp-2 was used.

Have been used textile materials from polypropylene fiber (PP-fiber), polyester fibers (PET-fiber) viscose fibers (Viscose-fiber).

The results obtained are presented in Figure 3.

## Examples 15-16

In these examples is presented the influence of the type of biopolymer used for the preparation of composite material according to the invention on the absorbency of a textile material using the preparation technology for the composite from Example 1and the methodology of impregnation of the textile material from Example 3.

Instead of gelatin have been used gum guar (G4129 Sigma Aldrich) and soluble starch (S9765 Sigma Aldrich).

Table 4 Influence of the biopolymer type (carbohydrates) on the absorbency of textile materials impregnated with polymeric composites containing synthetic polymer and biopolymer

| Example cod | Natural polymer type | Natural polymer in composite,% | IMD, % | $Q_2$-texic, (g/g) |
|---|---|---|---|---|
| Exp-15 | Guar | 1.8 | 19 | 16.96 |
| Exp-16 | Starch | 2.3 | 28 | 20.44 |

## Example 17

Table 5: Comparison of dry weight, absorbency in tap water and saline solution in nonwoven polypropylene (PP) and printed SAP on the same nonwoven PP

| | Nonwoven PP 22 [g/m$^2$] | | Printed SAP on nonwoven PP (175% add-on) | |
|---|---|---|---|---|
| | gr or gr/gr | Reference | gr or gr/gr | Add-on relative to Ref. |
| Dry weight [gr] | 0.4 | 100% | 1.1 | 275% |
| Absorbency tap water [gr/gr]* | 3 | 100% | 63 | 2100% |
| Absorbency saline [gr/gr]* | 3 | 100% | 21 | 700% |
| *Measured according to EDANA NW5P 010.1.R0. Saline = 0.9% NaCl solution, tap water = San Benedett mineral water. This sample provides 21 times the absorbency in tap water and 7 times the absorbency in saline relative to the regular product (nonwoven polypropylene (PP)) | | | | |

The polymer composite solution comprises synthetic polymer in the form of salt (SP), natural polymer (NP), additives (A) and water (W).

The ratio between a textile material TEX (before treatment) to impregnation composition IC [ IC =SP+NP +A] is from 85:25 to 99:1% by dry weight.

The ratio between the synthetic polymer (SP) in salt form to natural polymer (NP) SP/ NP is from 70:30 to 95:5% by dry weight.

The ratio of additives (A) to polymers A/(SP+NP) is from 0.5 to 5% dry weight.

Water content in the composite polymer solution is from 79 to 99% by weight.

Further processing of the wet textile material depends of initial density of material and of impregnation degree so chosen. For example:

a) if the density of the textile material has been initially higher than 70 g/m$^2$ , then the wet textile material is first dried in a stream of hot air at temperature of 50-90°C, preferable at temperature of 55-85°C, and most preferable at temperature of 60-80°C, so the solid composite (textile + composite polymer) resulted to have a humidity content less than 12% by weight, preferable less than 10% by weight, and most preferable less than 8% by weight and then the supplementary thermal treatment in hot air steam at temperature of 90-180°C, preferable at temperature of 100-170°C, and most preferable at temperature of 110-160°C, during a period of 5-180 minutes, preferable 10-150 minutes, and most preferable of 15-120 minutes;

b) if the density of textile material has been initially less than 70 $g/m^2$ the wet textile material is subdue to a single thermal treatment at temperature of 100-150°C, during 180-300 seconds, preferable at temperature of 140-180°C during 60-180 seconds and more preferable at temperature of 200-250°C during 30-120 seconds.

Following the thermal treatment the textile material is let to cool at room temperature and in the end is packed.

Reference to known method steps, conventional methods steps, known methods or conventional methods is not in any way an admission that any aspect, description or embodiment of the present invention is disclosed, taught or suggested in the relevant art. be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

## Claims

1. A multi-layer absorbent product, comprising:

   - a first layer which is liquid permeable or liquid impermeable; and
   - a layer of:

     - nonwoven, woven or knitted textile material comprising a network of fibres, or
     - textured plastic yarn comprising a network of surface pores,

   said layer of textile material or textured plastic yarn:

     - being disposed next to the first layer,
     - having a top surface with uniformly spaced super absorbent polymer (SAP) deposits of a synthetic polymer having monomers bearing carboxylic acid or carboxylic acid anhydride groups crosslinked to a natural polymer having amino and/or hydroxyl groups capable of crosslinking to COOH groups in the synthetic polymer in the absence of non-polymeric crosslinking agent on said network of fibres or surface pores, and
     - said top surface with uniformly spaced deposits being disposed against a surface of the first layer,

   wherein:

     - said layer of textured plastic yarn has a porous surface created by non-penetrating holes, indentations, depressions, open bubbles, and other roughness-creating features formed by foaming, etching, cutting or void-shaping methods;
     - the textured plastic yarn or nonwoven, woven or knitted textile material prior to depositing said SAP deposits has a density of between 20 to 150 $g/m^2$;
     - the weight of the SAP deposits relative to the textile material or textured plastic yarn is in the range of 100% to 600%;
     - said SAP deposits printed on the textile material or textured plastic yarn as substrate are uniformly spaced with a spacing in a range of 1 to 7 mm;
     - said SAP deposits of natural polymer crosslinked to synthetic polymer form a continuous SAP crosslinked polymer network that covers the fibres or pore walls underneath the SAP deposits and interpenetrates the network of fibres or network of pores acting as a scaffold to interconnect the SAP crosslinked polymer network with the fibre or pore network, with 10% $\pm$ 1% of each of said SAP deposit interpenetrating the network of fibres or network of pores and the 90% $\pm$ 1% remainder of each of said SAP deposit stuck to the upper porous surface of the textile material or textured plastic yarn, and
     - said layer of (i) nonwoven, woven or knitted textile material or (ii) textured plastic yarn with said SAP deposits have a water absorbency at least 10% higher than the absorbency of the same layer without said SAP deposits.

**2.** The multi-layer absorbent product of claim 1, wherein

- said layer of textile material or textured plastic yarn having said deposits is capable of absorbing at least 2 grams water per gram (g/g; gr/gr) of said layer, preferably at least 5 gr/gr, more preferably at least 10 gr/gr, more preferably at least 20 gr/gr, more preferably at least 30 gr/gr, even more preferably at least 40 gr/gr, still more preferably at least 60 gr/gr, still more preferably at least 80 gr/gr, most preferably at least 100 gr/gr, and/or
- the weight of said deposits relative to the textile material or textured plastic yarn is in a range of 200% to 600%, preferably 400% to 600%, and/or
- said deposits are uniformly spaced on the layer of textile material or textured plastic yarn, with the spacing in a range of preferably 1 to 6 mm, preferably about 2 mm, and/or
- said deposits are in the shape of circular dots or rectangles on the textile material or textured plastic yarn.

**3.** The multi-layer absorbent product of any of the preceding claims, wherein the synthetic polymer is:

- selected from linear or branched graft homo- or copolymers made from vinyl acidic monomers such as acrylic acid, maleic anhydride, itaconic anhydride and similar, optionally in association with other types of vinylic monomers that do not necessarily contain carboxylic acid functions, and/or
- a copolymer based on maleic anhydride and/or maleic acid, preferably a copolymer of styrene maleic anhydride (SMA), copolymer of isobutylene and maleic anhydride or copolymer of methyl vinyl ether and maleic acid.

**4.** The multi-layer absorbent product of any of the preceding claims, wherein the natural polymer:

- is a biopolymer selected from polysaccharides such as cellulose, alginate, dextran, chitosan, and the like; polyesters and lignin; guar; starch; water soluble phospholipids such as lecithin; polypeptides or proteins such as gelatine, albumin, soybean protein, collagen, collagenic biopolymer, collagen hydrolysate, and casein; and/or
- has amino and/or hydroxyl groups capable of cross-linking to COOH groups in said synthetic polymer under high temperature conditions and for selected periods of time.

**5.** The multi-layer absorbent product of any of the preceding claims, wherein said layer is a layer of nonwoven, woven or knitted textile material comprising a network of fibres, preferably

- said layer of nonwoven, woven or knitted textile material with said deposits has a water absorbency at least 20%, at least 30%, at least 50%, at least 80%, or at least 100% higher than the absorbency of the same layer without said deposits, more preferably at least 3 times higher or at least 4 times higher, most preferably at least 5 times higher, and/or
- said layer of nonwoven, woven or knitted textile material without said deposits has a density between preferably 20 to 60 g/m$^2$, more preferably 20 to 30 g/m$^2$, and/or
- the textile material and network of fibres are polypropylene.

**6.** The multi-layer absorbent product of claim 5, the first layer being a liquid permeable layer, wherein the liquid permeable layer is a second layer of said nonwoven, woven or knitted textile material having a top surface with said uniformly spaced deposits, and wherein the top surface of said second layer is disposed against the top surface of said first layer of said nonwoven, woven or knitted textile material such that the uniformly spaced SAP deposits on the top surfaces of said first and second layers do not overlap.

**7.** The multi-layer absorbent product of claim 5 or 6, the second layer being nonwoven, woven or knitted textile material comprising a network of fibres, the multi-layer absorbent product further comprising one or more liquid permeable layers, at least one of said one or more liquid permeable layers preferably being a further layer of said nonwoven, woven or knitted textile material having a top surface with said uniformly spaced SAP deposits.

**8.** The multi-layer absorbent product of any of claims 5 - 7, for use in absorbent products selected from diapers, cleaning wipes, household or institutional cleaning or maintenance appliances, hand wipes, hand towels, personal, cosmetic or sanitary wipes, baby wipes, facial tissues, hygienic absorbent pads, panties, panty liners, wound dressings and the like.

**9.** The multi-layer absorbent product of any of claims 1-4, wherein said layer is a layer of textured plastic yarn comprising a network of surface pores, preferably

- said layer of textured plastic yarn without said deposits has a density between 18 and 150 gr/m$^2$, more preferably 18 to 77 gr/m$^2$, and/or
- said layer of textured plastic yarn without said deposits has a thickness between 20 to 200 micrometres, more preferably 20 to 80 micrometres, and/or
- the plastic yarn and network of pore walls are polyethylene.

10. The multi-layer absorbent product of claim 9, the second layer being a textured plastic yarn comprising a network of surface pores layer,

- said first layer is a layer permeable for water vapor but not for liquids, and/or
- said first layer is a liquid impermeable layer, perforated with holes that confer on the layer a certain degree of water vapor and liquid water permeability, and/or
- further comprising one or more liquid impermeable layers, at least one of said one or more liquid impermeable layers preferably being a further layer of said textured plastic yarn having a top surface with said uniformly spaced SAP deposits.

11. The multi-layer absorbent product of claim 9 or 10 for use in the outdoor coverage or packing or packaging of vegetal agricultural crops such as fodder, hay or cotton, or crop residues such as straw, and their protection from rain, snow or ground moisture and the resultant damage from such uncontrolled humidity, to allow for their storage outdoors.

12. A process for preparing a layer of nonwoven, woven or knitted textile material with uniformly spaced deposits of a natural polymer crosslinked to a synthetic polymer in the multi-layer absorbent product of claim 5, comprising:

- depositing on a nonwoven, woven or knitted textile material, which has a network of fibers, uniformly spaced deposits of a polymeric composite aqueous solution in the form of a syrup for use in improving water absorbency of the textile material,

- the polymeric composite solution comprising a synthetic polymer having monomers bearing carboxylic acid or carboxylic acid anhydride groups and a natural polymer having amino and/or hydroxyl groups capable of crosslinking to COOH groups in the synthetic polymer, in which the natural and synthetic polymers in the composite solution are capable of undergoing self-crosslinking under controlled conditions of temperature and time and in the absence of non-polymeric crosslinking agent, wherein the concentration of the combination of natural and synthetic polymers is in a range of 20 - 40% w/w of the polymeric composite solution, wherein the nonwoven, woven or knitted textile material, prior to depositing, has a density of between 20 to 150 g/m$^2$; and

- thermally treating the textile material having uniformly spaced deposits thereon under controlled conditions of temperature and time so that the natural and synthetic polymers in the deposit undergo self-crosslinking in the absence of non-polymeric crosslinking agent in the deposits to form a continuous SAP crosslinked polymer network that covers the fibers underneath the SAP deposits and interpenetrates the network of fibers acting as a scaffold to interconnect the SAP crosslinked polymer network with the fiber network, with about 10% ± 1% of each of the SAP deposit interpenetrating the network of fibers and the about 90% ± 1% remainder of each of the SAP deposit stuck to the upper porous surface of the textile material, wherein the weight of the SAP deposits relative to the textile material is in the range of 100% to 600% and the SAP deposits printed on the textile material as substrate are uniformly spaced with a spacing in a range of 1 to 7 mm.

13. The process of claim 12,

- wherein in the depositing step, the uniformly spaced deposits are deposited on a continuous roll of nonwoven, woven or knitted textile material by rotary screen printing with a perforated cylinder, and from which individual sheets of the nonwoven, woven or knitted textile material with uniformly spaced SAP deposits thereon are later cut, preferably
- further comprising, after the thermal treating step, a step of cutting individual sheets of nonwoven, woven or knitted textile material with uniformly spaced SAP deposits from the continuous roll, and/or
- the nonwoven, woven or knitted textile material, prior to the depositing step, has a density between preferably 20 to 60 g/m$^2$, more preferably 20 to 30 g/m$^2$, and/or
- after being thermally treated, the weight of the deposits relative to the textile material is in a range of preferably 200% to 600%, more preferably 400% to 600%, and/or

- wherein the deposits are uniformly spaced on the nonwoven, woven or knitted textile material, with the spacing in a range of preferably 1 to 6 mm, more preferably about 2 mm, and/or
- wherein the textile material and network of fibres is polypropylene, and/or
- wherein the SAP deposits are in the shape of circular dots or squares on the textile material, and/or
- wherein the synthetic polymer is selected from linear or branched graft homo- or copolymers made from vinyl acidic monomers such as acrylic acid, maleic anhydride, itaconic anhydride and similar, optionally in association with other types of vinylic monomers that do not necessarily contain carboxylic acid functions, and/or
- wherein the synthetic polymer is a copolymer based on maleic anhydride and/or maleic acid, preferably a copolymer of styrene maleic anhydride (SMA), copolymer of isobutylene and maleic anhydride or copolymer of methyl vinyl ether and maleic acid, and/or
- wherein the natural polymer is a biopolymer selected from polysaccharides such as cellulose, alginate, dextran, chitosan, and the like; polyesters and lignin; guar; starch; water soluble phospholipids such as lecithin; polypeptides or proteins such as gelatine, albumin, soybean protein, collagen, collagenic biopolymer, collagen hydrolysate, and casein.

14. A process for preparing a layer of textured plastic yarn with uniformly spaced deposits of a natural polymer crosslinked to a synthetic polymer in the multi-layer absorbent product of claim 9, comprising:

- depositing on a textured plastic yarn, which has a surface network of pores, uniformly spaced deposits of a polymeric composite aqueous solution in the form of a syrup for use in improving water absorbency of the textured plastic yarn,

  - the polymeric composite solution comprising a synthetic polymer having monomers bearing carboxylic acid or carboxylic acid anhydride groups and a natural polymer having amino and/or hydroxyl groups capable of crosslinking to COOH groups in the synthetic polymer, in which the natural and synthetic polymers in the composite solution are capable of undergoing self-crosslinking under controlled conditions of temperature and time and in the absence of non-polymeric crosslinking agent, wherein the concentration of the combination of natural and synthetic polymers is in a range of 20 - 40% w/w of the polymeric composite solution, wherein the textured plastic yarn has a porous surface created by non-penetrating holes, indentations, depressions, open bubbles, and other roughness-creating features formed by foaming, etching, cutting or void-shaping methods, and prior to depositing, has a density of between 20 to 150 $g/m^2$; and

- thermally treating the textured plastic yarn having uniformly spaced deposits thereon under controlled conditions of temperature and time so that the natural and synthetic polymers in the deposit undergo self-crosslinking in the absence of non-polymeric crosslinking agent in the deposits to form a continuous SAP crosslinked polymer network that covers the pore walls underneath the SAP deposits and interpenetrates the network of pores acting as a scaffold to interconnect the SAP crosslinked polymer network with the pore network, with about 10% $\pm$ 1% of each of said SAP deposit interpenetrating the network of pores and the about 90% $\pm$ 1% remainder of each of said SAP deposit stuck to the upper porous surface of the textured plastic yarn, wherein the weight of the SAP deposits relative to the textured plastic yarn is in the range of 100% to 600% and the SAP deposits printed on the textured plastic yarn as substrate are uniformly spaced with a spacing in a range of 1 to 7 mm.

15. The process of claim 14,

- wherein, in the depositing step, the uniformly spaced deposits are deposited on a continuous roll of textured plastic yarn by rotary screen printing with a perforated cylinder, and from which individual sheets of the textured plastic yarn with uniformly spaced SAP deposits thereon are later cut, preferably
- further comprising, after the thermal treating step, a step of cutting individual sheets of textured plastic yarn with uniformly spaced SAP deposits from the continuous roll, and/or
- wherein, after being thermally treated, the weight of the deposits relative to the plastic yarn is in a range of preferably 200% to 600%, more preferably 400% to 600%, and/or
- wherein the deposits are uniformly spaced on the textured plastic yarn, with the spacing in a range of preferably 1 to 6 mm, more preferably about 2 mm, and/or
- wherein the textured plastic yarn and network of pore walls is polyethylene, and/or
- wherein the SAP deposits are in the shape of circular dots or squares on the textured plastic yarn, and/or
- wherein the synthetic polymer is preferably selected from linear or branched graft homo- or copolymers made from vinyl acidic monomers such as acrylic acid, maleic anhydride, itaconic anhydride and similar, optionally in association with other types of vinylic monomers that do not necessarily contain carboxylic acid functions, and/or

- wherein the synthetic polymer is a copolymer based on maleic anhydride and/or maleic acid, preferably a copolymer of styrene maleic anhydride (SMA), copolymer of isobutylene and maleic anhydride or copolymer of methyl vinyl ether and maleic acid, and/or wherein the natural polymer is a biopolymer selected from polysaccharides such as cellulose, alginate, dextran, chitosan, and the like; polyesters and lignin; guar; starch; water soluble phospholipids such as lecithin; polypeptides or proteins such as gelatine, albumin, soybean protein, collagen, collagenic biopolymer, collagen hydrolysate, and casein.

**Patentansprüche**

1. Ein mehrschichtiges absorbierendes Produkt, umfassend:

   - eine erste Schicht, die flüssigkeitsdurchlässig oder flüssigkeitsundurchlässig ist; und
   - eine Schicht aus:

     - nichtgewebtem, gewebtem oder gewirktem Textilmaterial, das ein Netzwerk von Fasern umfasst, oder
     - texturiertem Kunststoffgarn mit einem Netz von Oberflächenporen,

   wobei die Schicht des textilen Materials oder des texturierten Kunststoffgarns:

     - neben der ersten Schicht abgelagert ist,
     - eine obere Oberfläche mit gleichmäßig beabstandeten superabsorbierenden Polymer (SAP)-Ablagerungen aus einem synthetischen Polymer mit Monomeren, die Carbonsäure- oder Carbonsäureanhydridgruppen tragen, die mit einem natürlichen Polymer vernetzt sind, das Amino- und/oder Hydroxylgruppen aufweist, die in der Lage sind, mit COOH-Gruppen in dem synthetischen Polymer in Abwesenheit eines nichtpolymeren Vernetzungsmittels auf dem Fasernetz oder den Oberflächenporen zu vernetzen, und
     - die obere Oberfläche mit gleichmäßig beabstandeten Ablagerungen, die gegen eine Oberfläche der ersten Schicht angeordnet sind,

   wobei:

     - die Schicht aus texturiertem Kunststoffgarn eine poröse Oberfläche aufweist, die durch nicht durchdringende Löcher, Einkerbungen, Vertiefungen, offene Blasen und andere Rauhigkeitsmerkmale erzeugt wird, die durch Aufschäumen, Ätzen, Schneiden oder Hohlraumformungsverfahren gebildet werden;
     - das texturierte Kunststoffgarn oder das nichtgewebte, gewebte oder gewirkte Textilmaterial vor dem Aufbringen der SAP-Ablagerungen eine Dichte zwischen 20 und 150 g/m² aufweist;
     - das Gewicht der SAP-Ablagerungen im Verhältnis zum textilen Material oder texturierten Kunststoffgarn im Bereich von 100% bis 600% liegt;
     - die SAP-Ablagerungen, die auf das Textilmaterial oder das texturierte Kunststoffgarn als Substrat gedruckt sind, gleichmäßig mit einem Abstand im Bereich von 1 bis 7 mm angeordnet sind;
     - die SAP-Ablagerungen aus natürlichem Polymer, das mit synthetischem Polymer vernetzt ist, ein kontinuierliches SAP-vernetztes Polymernetzwerk bilden, das die Fasern oder Porenwände unterhalb der SAP-Ablagerungen bedeckt und das Fasernetzwerk oder das Porennetzwerk durchdringt und als Gerüst dient, um das SAP-vernetzte Polymernetzwerk mit dem Faser- oder Porennetzwerk zu verbinden, wobei 10% ± 1% jeder SAP-Ablagerung das Fasernetzwerk oder das Porennetzwerk durchdringt und die restlichen 90% ± 1% jeder SAP-Ablagerung an der oberen porösen Oberfläche des Textilmaterials oder des texturierten Kunststoffgarns haften, und
     - die Schicht aus (i) nichtgewebtem, gewebtem oder gewirktem Textilmaterial oder (ii) texturiertem Kunststoffgarn mit den SAP-Ablagerungen eine Wasserabsorptionsfähigkeit aufweist, die mindestens 10 % höher ist als die Absorptionsfähigkeit der gleichen Schicht ohne die SAP-Ablagerungen.

2. Das mehrschichtige absorbierende Produkt nach Anspruch 1, wobei

   - die Schicht aus textilem Material oder texturiertem Kunststoffgarn mit den Einlagerungen in der Lage ist, mindestens 2 Gramm Wasser pro Gramm (g/g; gr/gr) der Schicht zu absorbieren, vorzugsweise mindestens 5 gr/gr, noch bevorzugter mindestens 10 gr/gr, noch bevorzugter mindestens 20 gr/gr, noch bevorzugter mindestens 30 gr/gr, noch bevorzugter mindestens 40 gr/gr, noch bevorzugter mindestens 60 gr/gr, noch bevorzugter mindestens 80 gr/gr, am meisten bevorzugt mindestens 100 gr/gr, und/oder

- das Gewicht der Ablagerungen in Bezug auf das Textilmaterial oder das texturierte Kunststoffgarn im Bereich von 200% bis 600%, vorzugsweise 400% bis 600%, liegt und/oder
- die Ablagerungen gleichmäßig auf der Schicht aus textilem Material oder texturiertem Kunststoffgarn verteilt sind, wobei der Abstand in einem Bereich von vorzugsweise 1 bis 6 mm, vorzugsweise etwa 2 mm, liegt, und/oder
- die Ablagerungen die Form von kreisförmigen Punkten oder Rechtecken auf dem textilen Material oder dem texturierten Kunststoffgarn haben.

3. Das mehrschichtige absorbierende Produkt nach einem der vorhergehenden Ansprüche, wobei das synthetische Polymer ist:

- ausgewählt aus linearen oder verzweigten Pfropfhomo- oder -copolymeren aus vinylsauren Monomeren wie Acrylsäure, Maleinsäureanhydrid, Itaconsäureanhydrid und ähnlichen, gegebenenfalls in Verbindung mit anderen Arten von vinylischen Monomeren, die nicht unbedingt Carbonsäurefunktionen enthalten, und/oder
- ein Copolymer auf Basis von Maleinsäureanhydrid und/oder Maleinsäure, vorzugsweise ein Copolymer aus Styrol-Maleinsäureanhydrid (SMA), ein Copolymer aus Isobutylen und Maleinsäureanhydrid oder ein Copolymer aus Methylvinylether und Maleinsäure.

4. Das mehrschichtige absorbierende Produkt nach einem der vorhergehenden Ansprüche, wobei das natürliche Polymer:

- ein Biopolymer ist, ausgewählt aus Polysacchariden wie Cellulose, Alginat, Dextran, Chitosan und dergleichen; Polyestern und Lignin; Guar; Stärke; wasserlöslichen Phospholipiden wie Lecithin; Polypeptiden oder Proteinen wie Gelatine, Albumin, Sojaprotein, Kollagen, kollagenem Biopolymer, Kollagenhydrolysat und Casein; und/oder
- Amino- und/oder Hydroxylgruppen aufweist, die in der Lage sind, sich unter Hochtemperaturbedingungen und für ausgewählte Zeiträume mit COOH-Gruppen in dem synthetischen Polymer zu vernetzen.

5. Das mehrschichtige absorbierende Produkt nach einem der vorhergehenden Ansprüche, wobei die Schicht eine Schicht aus einem nicht gewebten, gewebten oder gewirkten Textilmaterial ist, das ein Netzwerk von Fasern umfasst, wobei vorzugsweise

- die Schicht aus nichtgewebtem, gewebtem oder gewirktem Textilmaterial mit den Ablagerungen eine Wasserabsorptionsfähigkeit aufweist, die mindestens 20%, mindestens 30%, mindestens 50%, mindestens 80% oder mindestens 100% höher ist als die Absorptionsfähigkeit der gleichen Schicht ohne die Ablagerungen, vorzugsweise mindestens 3-mal höher oder mindestens 4-mal höher, am meisten bevorzugt mindestens 5-mal höher, und/oder
- die Schicht aus Vliesstoff, gewebtem oder gewirktem Textilmaterial ohne die Ablagerungen eine Dichte von vorzugsweise 20 bis 60 g/m$^2$, besonders bevorzugt 20 bis 30 g/m$^2$ aufweist, und/oder
- das textile Material und das Fasernetz aus Polypropylen bestehen.

6. Mehrschichtiges absorbierendes Produkt nach Anspruch 5, wobei die erste Schicht eine flüssigkeitsdurchlässige Schicht ist, wobei die flüssigkeitsdurchlässige Schicht eine zweite Schicht des nicht gewebten, gewebten oder gewirkten Textilmaterials ist, die eine obere Oberfläche mit den gleichmäßig beabstandeten Ablagerungen aufweist, und wobei die obere Oberfläche der zweiten Schicht gegen die obere Oberfläche der ersten Schicht des nicht gewebten, gewebten oder gewirkten Textilmaterials so angeordnet ist, dass sich die gleichmäßig beabstandeten SAP-Ablagerungen auf den oberen Oberflächen der ersten und zweiten Schicht nicht überlappen.

7. Mehrschichtiges absorbierendes Produkt nach Anspruch 5 oder 6, wobei die zweite Schicht ein nichtgewebtes, gewebtes oder gewirktes Textilmaterial ist, das ein Netzwerk von Fasern umfasst, wobei das mehrschichtige absorbierende Produkt weiterhin eine oder mehrere flüssigkeitsdurchlässige Schichten umfasst, wobei mindestens eine der einen oder mehreren flüssigkeitsdurchlässigen Schichten vorzugsweise eine weitere Schicht des nichtgewebten, gewebten oder gewirkten Textilmaterials ist, die eine obere Oberfläche mit den gleichmäßig beabstandeten SAP-Einlagerungen aufweist.

8. Das mehrschichtige absorbierende Produkt nach einem der Ansprüche 5 bis 7 zur Verwendung in absorbierenden Produkten, ausgewählt aus Windeln, Reinigungstüchern, Haushalts- oder institutionellen Reinigungs- oder Pflegegeräten, Handtüchern, Handtüchern, Tüchern für den persönlichen Gebrauch, Kosmetiktüchern oder Hygienetüchern, Babytüchern, Gesichtstüchern, hygienischen Saugkissen, Höschen, Slipeinlagen, Wundverbänden und dergleichen.

9. Das mehrschichtige absorbierende Produkt nach einem der Ansprüche 1 bis 4, wobei die Schicht eine Schicht aus texturiertem Kunststoffgarn ist, das ein Netzwerk von Oberflächenporen aufweist, wobei vorzugsweise

   - die Schicht aus texturiertem Kunststoffgarn ohne die Ablagerungen eine Dichte zwischen 18 und 150 gr/m$^2$, vorzugsweise 18 bis 77 gr/m$^2$, aufweist und/oder
   - die Schicht aus texturiertem Kunststoffgarn ohne die Ablagerungen eine Dicke zwischen 20 und 200 Mikrometern, vorzugsweise zwischen 20 und 80 Mikrometern, aufweist, und/oder
   - das Kunststoffgarn und das Netz der Porenwände aus Polyethylen bestehen.

10. Mehrschichtiges saugfähiges Produkt nach Anspruch 9, wobei die zweite Schicht ein texturiertes Kunststoffgarn ist, das eine Schicht mit einem Netzwerk von Oberflächenporen umfasst, wobei

    - die erste Schicht eine Schicht ist, die für Wasserdampf, aber nicht für Flüssigkeiten durchlässig ist, und/oder
    - die erste Schicht eine flüssigkeitsundurchlässige Schicht ist, die mit Löchern perforiert ist, die der Schicht einen gewissen Grad an Wasserdampf- und Flüssigwasserdurchlässigkeit verleihen, und/oder
    - die zweite Schicht ferner eine oder mehrere flüssigkeitsundurchlässige Schichten umfasst, wobei mindestens eine der einen oder mehreren flüssigkeitsundurchlässigen Schichten vorzugsweise eine weitere Schicht des texturierten Kunststoffgarns mit einer Oberseite mit den gleichmäßig beabstandeten SAP-Einlagerungen ist.

11. Mehrschichtiges absorbierendes Produkt nach Anspruch 9 oder 10 zur Verwendung bei der Abdeckung oder Verpackung von pflanzlichen landwirtschaftlichen Erzeugnissen wie Futter, Heu oder Baumwolle oder Ernterückständen wie Stroh im Freien und zu deren Schutz vor Regen, Schnee oder Bodenfeuchtigkeit und den sich daraus ergebenden Schäden durch diese unkontrollierte Feuchtigkeit, um ihre Lagerung im Freien zu ermöglichen.

12. Verfahren zur Herstellung einer Schicht aus nichtgewebtem, gewebtem oder gewirktem Textilmaterial mit gleichmäßig beabstandeten Ablagerungen eines natürlichen Polymers, das mit einem synthetischen Polymer vernetzt ist, in dem mehrlagigen absorbierenden Produkt nach Anspruch 5, umfassend:

    - Aufbringen von gleichmäßig beabstandeten Ablagerungen einer wässrigen polymeren Verbundlösung in Form eines Sirups auf ein nicht gewebtes, gewobenes oder gewirktes Textilmaterial, das ein Netzwerk von Fasern aufweist, zur Verwendung bei der Verbesserung der Wasserabsorptionsfähigkeit des Textilmaterials,

      - wobei die polymere Verbundlösung ein synthetisches Polymer mit Monomeren, die Carbonsäure- oder Carbonsäureanhydridgruppen tragen, und ein natürliches Polymer mit Amino- und/oder Hydroxylgruppen, die in der Lage sind, mit COOH-Gruppen in dem synthetischen Polymer zu vernetzen, umfasst, wobei die natürlichen und synthetischen Polymere in der Verbundlösung in der Lage sind, sich unter kontrollierten Temperatur- und Zeitbedingungen und in Abwesenheit eines nichtpolymeren Vernetzungsmittels selbst zu vernetzen, wobei die Konzentration der Kombination aus natürlichen und synthetischen Polymeren in einem Bereich von 20 bis 40 Gew.-% der polymeren Verbundlösung liegt, wobei das nichtgewebte, gewebte oder gewirkte Textilmaterial vor dem Ablegen eine Dichte zwischen 20 und 150 g/m$^2$ aufweist; und

      - thermische Behandlung des Textilmaterials mit gleichmäßig beabstandeten Ablagerungen darauf unter kontrollierten Temperatur- und Zeitbedingungen, so dass die natürlichen und synthetischen Polymere in der Ablagerung in Abwesenheit eines nichtpolymeren Vernetzungsmittels in den Ablagerungen selbstvernetzend werden, um ein kontinuierliches SAP-vernetztes Polymernetzwerk zu bilden, das die Fasern unter den SAP-Ablagerungen bedeckt und das Netzwerk von Fasern durchdringt, die als Gerüst wirken, um das SAP-vernetzte Polymernetzwerk mit dem Fasernetzwerk zu verbinden, wobei etwa 10% ± 1% jeder der SAP-Ablagerungen das Fasernetz durchdringen und der etwa 90% ± 1%ige Rest jeder der SAP-Ablagerungen an der oberen porösen Oberfläche des Textilmaterials haftet, wobei das Gewicht der SAP-Ablagerungen relativ zum Textilmaterial im Bereich von 100% bis 600% liegt und die auf das Textilmaterial als Substrat gedruckten SAP-Ablagerungen gleichmäßig mit einem Abstand im Bereich von 1 bis 7 mm beabstandet sind.

13. Das Verfahren nach Anspruch 12,

    - wobei im Abscheidungsschritt die gleichmäßig beabstandeten Abscheidungen durch Rotationssiebdruck mit einem perforierten Zylinder auf eine Endlosrolle von nichtgewebtem, gewebtem oder gewirktem Textilmaterial aufgebracht werden, von der später einzelne Blätter des nichtgewebten, gewebten oder gewirkten Textilmaterials mit gleichmäßig beabstandeten SAP-Abscheidungen darauf geschnitten werden, wobei vorzugsweise

- das Verfahren ferner nach dem thermischen Behandlungsschritt einen Schritt des Schneidens einzelner Bahnen des nicht gewebten, gewebten oder gewirkten Textilmaterials mit gleichmäßig beabstandeten SAP-Einlagerungen von der Endlosrolle umfasst, und/oder

- das nichtgewebte, gewebte oder gewirkte Textilmaterial vor dem Ablagerungsschritt eine Dichte zwischen vorzugsweise 20 bis 60 g/m$^2$, mehr bevorzugt 20 bis 30 g/m$^2$, aufweist, und/oder

- nach der Wärmebehandlung das Gewicht der Ablagerungen im Verhältnis zum Textilmaterial in einem Bereich von vorzugsweise 200% bis 600%, mehr bevorzugt 400% bis 600%, liegt, und/oder

- die Ablagerungen gleichmäßig auf dem nicht gewebten, gewebten oder gewirkten Textilmaterial verteilt sind, wobei der Abstand in einem Bereich von vorzugsweise 1 bis 6 mm, besonders bevorzugt etwa 2 mm, liegt, und/oder

- das textile Material und das Fasernetz aus Polypropylen besteht, und/oder

- die SAP-Ablagerungen in Form von kreisförmigen Punkten oder Quadraten auf dem Textilmaterial vorliegen, und/oder

- das synthetische Polymer aus linearen oder verzweigten Pfropfhomo- oder -copolymeren ausgewählt ist, die aus vinylsauren Monomeren wie Acrylsäure, Maleinsäureanhydrid, Itaconsäureanhydrid und ähnlichen hergestellt sind, gegebenenfalls in Verbindung mit anderen Arten von Vinylmonomeren, die nicht unbedingt Carbonsäurefunktionen enthalten, und/oder

- das synthetische Polymer ein Copolymer auf Basis von Maleinsäureanhydrid und/oder Maleinsäure ist, vorzugsweise ein Copolymer aus Styrol-Maleinsäureanhydrid (SMA), ein Copolymer aus Isobutylen und Maleinsäureanhydrid oder ein Copolymer aus Methylvinylether und Maleinsäure, und/oder

- das natürliche Polymer ein Biopolymer ist, das aus Polysacchariden wie Cellulose, Alginat, Dextran, Chitosan und dergleichen, Polyestern und Lignin, Guar, Stärke, wasserlöslichen Phospholipiden wie Lecithin, Polypeptiden oder Proteinen wie Gelatine, Albumin, Sojaprotein, Kollagen, kollagenem Biopolymer, Kollagenhydrolysat und Kasein ausgewählt ist.

14. Verfahren zur Herstellung einer Schicht aus texturiertem Kunststoffgarn mit gleichmäßig beabstandeten Ablagerungen eines natürlichen Polymers, das mit einem synthetischen Polymer vernetzt ist, in dem mehrschichtigen absorbierenden Produkt nach Anspruch 9, umfassend:

- Aufbringen von gleichmäßig beabstandeten Ablagerungen einer polymeren wässrigen Verbundlösung in Form eines Sirups auf ein texturiertes Kunststoffgarn, das ein Oberflächennetzwerk von Poren aufweist, zur Verwendung bei der Verbesserung der Wasserabsorptionsfähigkeit des texturierten Kunststoffgarns,

- wobei die polymere Verbundlösung, ein synthetisches Polymer mit Monomeren, die Carbonsäure- oder Carbonsäureanhydridgruppen tragen, und ein natürliches Polymer mit Amino- und/oder Hydroxylgruppen, die zur Vernetzung mit COOH-Gruppen in dem synthetischen Polymer fähig sind, umfasst, wobei die natürlichen und synthetischen Polymere in der Verbundlösung in der Lage sind, unter kontrollierten Temperatur- und Zeitbedingungen und in Abwesenheit eines nichtpolymeren Vernetzungsmittels selbstvernetzend zu sein, wobei die Konzentration der Kombination aus natürlichen und synthetischen Polymeren in einem Bereich von 20 - 40 % (w/w) der polymeren Verbundlösung liegt, wobei das texturierte Kunststoffgarn eine poröse Oberfläche aufweist, die durch nicht durchdringende Löcher, Vertiefungen, Eindrücke, offene Blasen und andere Rauheit erzeugende Merkmale erzeugt wird, die durch Schäum-, Ätz-, Schneid- oder Hohlraumformungsverfahren gebildet werden, und vor dem Ablegen eine Dichte zwischen 20 und 150 g/m2 aufweist; und

- thermisch Behandeln des texturierten Kunststoffgarns mit darauf gleichmäßig beabstandeten Ablagerungen unter kontrollierten Temperatur- und Zeitbedingungen, so dass die natürlichen und synthetischen Polymere in der Ablagerung in Abwesenheit eines nichtpolymeren Vernetzungsmittels in den Ablagerungen selbstvernetzend werden, um ein kontinuierliches SAP-vernetztes Polymernetzwerk zu bilden, das die Porenwände unterhalb der SAP-Ablagerungen bedeckt und das Netzwerk von Poren durchdringt, die als Gerüst wirken, um das SAP-vernetzte Polymernetzwerk mit dem Porennetzwerk zu verbinden, wobei etwa 10% ± 1% jeder der SAP-Ablagerungen das Porennetzwerk durchdringen und der etwa 90% ± 1%ige Rest jeder der SAP-Ablagerungen an der oberen porösen Oberfläche des texturierten Kunststoffgarns haftet, wobei das Gewicht der SAP-Ablagerungen relativ zu dem texturierten Kunststoffgarn im Bereich von 100% bis 600% liegt und die auf das texturierte Kunststoffgarn als Substrat gedruckten SAP-Ablagerungen gleichmäßig mit einem Abstand im Bereich von 1 bis 7 mm beabstandet sind.

15. Verfahren nach Anspruch 14,

- wobei im Abscheidungsschritt die gleichmäßig beabstandeten Abscheidungen durch Rotationssiebdruck mit einem perforierten Zylinder auf eine Endlosrolle texturierten Kunststoffgarns aufgebracht werden, von der später einzelne Bögen des texturierten Kunststoffgarns mit gleichmäßig beabstandeten SAP-Abscheidungen darauf geschnitten werden, wobei vorzugsweise

- das Verfahren ferner nach dem thermischen Behandlungsschritt einen Schritt des Schneidens einzelner Blätter des texturierten Kunststoffgarns mit gleichmäßig beabstandeten SAP-Einlagerungen von der Endlosrolle umfasst, und/oder

- nach der Wärmebehandlung das Gewicht der Ablagerungen im Verhältnis zum Kunststoffgarn in einem Bereich von vorzugsweise 200% bis 600%, besonders bevorzugt 400% bis 600% liegt, und/oder

- die Ablagerungen gleichmäßig auf dem texturierten Kunststoffgarn verteilt sind, wobei der Abstand in einem Bereich von vorzugsweise 1 bis 6 mm, besonders bevorzugt etwa 2 mm, liegt, und/oder

- das texturierte Kunststoffgarn und das Netzwerk von Porenwänden aus Polyethylen besteht, und/oder

- die SAP-Ablagerungen in Form von kreisförmigen Punkten oder Quadraten auf dem texturierten Kunststoffgarn vorliegen, und/oder

- das synthetische Polymer vorzugsweise aus linearen oder verzweigten Pfropfhomo- oder - copolymeren ausgewählt ist, die aus vinylsauren Monomeren wie Acrylsäure, Maleinsäureanhydrid, Itaconsäureanhydrid und ähnlichen hergestellt sind, gegebenenfalls in Verbindung mit anderen Arten von vinylischen Monomeren, die nicht notwendigerweise Carbonsäurefunktionen enthalten, und/oder

- das synthetische Polymer ein Copolymer auf der Basis von Maleinsäureanhydrid und/oder Maleinsäure ist, vorzugsweise ein Copolymer aus Styrol-Maleinsäureanhydrid (SMA), ein Copolymer aus Isobutylen und Maleinsäureanhydrid oder ein Copolymer aus Methylvinylether und Maleinsäure, und/oder wobei das natürliche Polymer ein Biopolymer ist, das aus Polysacchariden wie Cellulose, Alginat, Dextran, Chitosan und dergleichen ausgewählt ist; Polyestern und Lignin; Guar; Stärke; wasserlöslichen Phospholipiden wie Lecithin; Polypeptiden oder Proteinen wie Gelatine, Albumin, Sojaprotein, Kollagen, kollagenem Biopolymer, Kollagenhydrolysat und Casein.

## Revendications

1. Produit absorbant multicouche, comprenant :

   - une première couche perméable ou imperméable aux liquides ; et
   - une couche de :

      - matière textile non tissée, tissée ou tricotée comprenant un réseau de fibres, ou
      - fil plastique texturé comprenant un réseau de pores superficiels,

   - ladite couche de matière textile ou de fil plastique texturé :

      - est disposée à côté de la première couche,
      - ayant une surface supérieure avec des dépôts uniformément espacés de polymère super absorbant (SAP) d'un polymère synthétique ayant des monomères portant des groupes d'acide carboxylique ou d'anhydride d'acide carboxylique réticulés à un polymère naturel ayant des groupes amino et/ou hydroxyle capables de réticuler à des groupes COOH dans le polymère synthétique en l'absence d'agent de réticulation non polymérique sur ledit réseau de fibres ou de pores de surface, et
      - ladite surface supérieure avec des dépôts uniformément espacés et disposés contre une surface de la première couche,

   dans laquelle :

   - ladite couche de fil plastique texturé présente une surface poreuse créée par des trous non pénétrants, des indentations, des dépressions, des bulles ouvertes et d'autres caractéristiques de rugosité formées par des méthodes de moussage, de gravure, de coupe ou de façonnage des vides ;
   - le fil plastique texturé ou le matériau textile non tissé, tissé ou tricoté avant le dépôt des dépôts SAP a une densité comprise entre 20 et 150 g/m$^2$ ;
   - le poids des dépôts SAP par rapport au matériau textile ou au fil plastique texturé est compris entre 100 % et 600 % ;
   - lesdits dépôts SAP imprimés sur le matériau textile ou le fil plastique texturé en tant que substrat sont uniformément espacés d'une distance comprise entre 1 et 7 mm ;

- lesdits dépôts SAP de polymère naturel réticulé à un polymère synthétique forment un réseau continu de polymère réticulé SAP qui recouvre les fibres ou les parois des pores sous les dépôts SAP et interpénètre le réseau de fibres ou le réseau de pores agissant comme un échafaudage pour interconnecter le réseau de polymère réticulé SAP avec le réseau de fibres ou de pores, avec 10 % $\pm$ 1 % de chacun desdits dépôts SAP interpénétrant le réseau de fibres ou le réseau de pores et le reste de 90 % $\pm$ 1 % de chacun desdits dépôts SAP collé à la surface poreuse supérieure de la matière textile ou du fil plastique texturé, et

- ladite couche (i) de matière textile non tissée, tissée ou tricotée ou (ii) de fil plastique texturé avec lesdits dépôts de SAP a un pouvoir d'absorption de l'eau supérieur d'au moins 10 % au pouvoir d'absorption de la même couche sans lesdits dépôts de SAP.

2. Le produit absorbant multicouche de la revendication 1, dans lequel

- ladite couche de matière textile ou de fil plastique texturé comportant lesdits dépôts est capable d'absorber au moins 2 grammes d'eau par gramme (g/g ; gr/gr) de ladite couche, de préférence au moins 5 gr/gr, de préférence au moins 10 gr/gr, de préférence au moins 20 gr/gr, de préférence au moins 30 gr/gr, de préférence encore au moins 40 gr/gr, de préférence encore au moins 60 gr/gr, de préférence encore au moins 80 gr/gr, de préférence encore au moins 100 gr/gr, et/ou

- le poids desdits dépôts par rapport à la matière textile ou au fil plastique texturé est compris entre 200% et 600%, de préférence entre 400% et 600%, et/ou

- lesdits dépôts sont uniformément espacés sur la couche de matière textile ou de fil plastique texturé, l'espacement étant de préférence compris entre 1 et 6 mm, de préférence environ 2 mm, et/ou

- lesdits dépôts ont la forme de points circulaires ou de rectangles sur la matière textile ou le fil plastique texturé.

3. Le produit absorbant multicouche de l'une quelconque des revendications précédentes, dans lequel le polymère synthétique est :

- choisi parmi les homo- ou copolymères greffés linéaires ou ramifiés fabriqués à partir de monomères vinylacides tels que l'acide acrylique, l'anhydride maléique, l'anhydride itaconique et similaires, éventuellement en association avec d'autres types de monomères vinyliques qui ne contiennent pas nécessairement de fonctions acide carboxylique, et/ou

- un copolymère à base d'anhydride maléique et/ou d'acide maléique, de préférence un copolymère d'anhydride styrène-maléique (SMA), un copolymère d'isobutylène et d'anhydride maléique ou un copolymère d'éther méthylvinylique et d'acide maléique.

4. Le produit absorbant multicouche de l'une des revendications précédentes, dans lequel le polymère naturel :

- est un biopolymère choisi parmi les polysaccharides tels que la cellulose, l'alginate, le dextrane, le chitosan et autres ; les polyesters et la lignine ; le guar ; l'amidon ; les phospholipides solubles dans l'eau tels que la lécithine ; les polypeptides ou les protéines tels que la gélatine, l'albumine, la protéine de soja, le collagène, le biopolymère collagénique, l'hydrolysat de collagène, et la caséine ; et / ou

- possède des groupes amino et/ou hydroxyle capables de se réticuler avec les groupes COOH dudit polymère synthétique dans des conditions de température élevée et pendant des périodes de temps déterminées.

5. Le produit absorbant multicouche de l'une quelconque des revendications précédentes, dans lequel ladite couche est une couche de matériau textile non tissé, tissé ou tricoté comprenant un réseau de fibres, de préférence

- ladite couche de matériau textile non tissé, tissé ou tricoté avec lesdits dépôts a un pouvoir absorbant d'au moins 20%, au moins 30%, au moins 50%, au moins 80%, ou au moins 100% plus élevé que le pouvoir absorbant de la même couche sans lesdits dépôts, plus préférentiellement au moins 3 fois plus élevé ou au moins 4 fois plus élevé, plus préférentiellement au moins 5 fois plus élevé, et/ou

- ladite couche de matière textile non tissée, tissée ou tricotée sans lesdits dépôts a une densité comprise de préférence entre 20 et 60 g/m$^2$, plus préférentiellement entre 20 et 30 g/m$^2$, et/ou

- le matériau textile et le réseau de fibres sont en polypropylène.

6. Le produit absorbant multicouche de la revendication 5, la première couche étant une couche perméable aux liquides, dans laquelle la couche perméable aux liquides est une deuxième couche dudit matériau textile non tissé, tissé ou tricoté ayant une surface supérieure avec lesdits dépôts uniformément espacés, et dans laquelle la surface supérieure de ladite deuxième couche est disposée contre la surface supérieure de ladite première couche dudit matériau textile non tissé, tissé ou tricoté de telle sorte que les dépôts SAP uniformément espacés sur les surfaces supérieures desdites première et deuxième couches ne se chevauchent pas.

7. Le produit absorbant multicouche de la revendication 5 ou 6, la deuxième couche étant un matériau textile non tissé, tissé ou tricoté comprenant un réseau de fibres, le produit absorbant multicouche comprenant en outre une ou plusieurs couches perméables aux liquides, au moins une de ces couches perméables aux liquides étant de préférence une autre couche dudit matériau textile non tissé, tissé ou tricoté ayant une surface supérieure avec lesdits dépôts SAP uniformément espacés.

8. Le produit absorbant multicouche de l'une des revendications 5 à 7, destiné à être utilisé dans des produits absorbants choisis parmi les couches, les lingettes nettoyantes, les appareils de nettoyage ou d'entretien ménager ou institutionnel, les essuie-mains, les essuie-mains, les lingettes personnelles, cosmétiques ou sanitaires, les lingettes pour bébés, les mouchoirs en papier, les tampons absorbants hygiéniques, les culottes, les protège-slips, les pansements et autres.

9. Le produit absorbant multicouche de l'une des revendications 1 à 4, dans lequel ladite couche est une couche de fil plastique texturé comprenant un réseau de pores de surface, de préférence

- ladite couche de fil plastique texturé sans lesdits dépôts a une densité comprise entre 18 et 150 gr/m$^2$, plus préférentiellement entre 18 et 77 gr/m$^2$, et/ou
- ladite couche de fil plastique texturé sans lesdits dépôts a une épaisseur comprise entre 20 et 200 micromètres, plus préférentiellement entre 20 et 80 micromètres, et/ou
- le fil plastique et le réseau de parois des pores sont en polyéthylène.

10. Le produit absorbant multicouche de la revendication 9, la deuxième couche étant un fil plastique texturé comprenant un réseau de pores de surface,

- ladite première couche est une couche perméable à la vapeur d'eau mais pas aux liquides, et/ou
- ladite première couche est une couche imperméable aux liquides, perforée de trous qui confèrent à la couche un certain degré de perméabilité à la vapeur d'eau et à l'eau liquide, et/ou
- comprenant en outre une ou plusieurs couches imperméables aux liquides, l'une au moins de ces couches imperméables aux liquides étant de préférence une autre couche dudit fil plastique texturé dont la surface supérieure présente lesdits dépôts SAP uniformément espacés.

11. Le produit absorbant multicouche de la revendication 9 ou 10 est utilisé pour couvrir, emballer ou conditionner à l'extérieur des cultures agricoles végétales telles que le fourrage, le foin ou le coton, ou des résidus de culture tels que la paille, et pour les protéger de la pluie, de la neige ou de l'humidité du sol et des dommages résultant d'une humidité incontrôlée, afin de permettre leur stockage à l'extérieur.

12. Procédé de préparation d'une couche de matériau textile non tissé, tissé ou tricoté avec des dépôts uniformément espacés d'un polymère naturel réticulé à un polymère synthétique dans le produit absorbant multicouche de la revendication 5, comprenant :

- déposer sur un matériau textile non tissé, tissé ou tricoté, qui a un réseau de fibres, des dépôts uniformément espacés d'une solution aqueuse composite polymère sous la forme d'un sirop pour l'utilisation dans l'amélioration de l'absorption d'eau du matériau textile,

- la solution composite polymère comprenant un polymère synthétique ayant des monomères portant des groupes acide carboxylique ou anhydride d'acide carboxylique et un polymère naturel ayant des groupes amino et/ou hydroxyle capables de se réticuler aux groupes COOH dans le polymère synthétique, dans laquelle les polymères naturels et synthétiques de la solution composite sont capables de subir une auto-réticulation dans des conditions contrôlées de température et de temps et en l'absence d'agent de réticulation non polymère, dans laquelle la concentration de la combinaison de polymères naturels et synthétiques est comprise entre 20 et 40 % p/p de la solution composite polymère, dans laquelle le matériau textile non tissé, tissé ou tricoté, avant le dépôt, a une densité comprise entre 20 et 150 g/m$^2$ ; et

- traiter thermiquement le matériau textile avec des dépôts uniformément espacés dans des conditions contrôlées de température et de temps afin que les polymères naturels et synthétiques dans le dépôt subissent une auto-réticulation en l'absence d'agent de réticulation non polymère dans les dépôts pour former un réseau polymère réticulé SAP continu qui recouvre les fibres sous les dépôts SAP et interpénètre le réseau de fibres agissant comme un échafaudage pour interconnecter le réseau polymère réticulé SAP avec le réseau de fibres, avec environ 10 % ± 1 % de chaque dépôt de SAP interpénétrant le réseau de fibres et le reste d'environ 90 % ± 1 % de chaque dépôt de SAP collé à la surface poreuse supérieure du matériau textile, dans lequel le poids des dépôts de SAP par rapport au matériau textile est compris entre 100 % et 600 % et les dépôts de SAP imprimés sur le matériau textile en tant que substrat sont uniformément espacés d'un espacement compris entre 1 et 7 mm.

**13.** Le procédé de la revendication 12,

- dans lequel, dans l'étape de dépôt, les dépôts uniformément espacés sont déposés sur un rouleau continu de matériau textile non tissé, tissé ou tricoté par sérigraphie rotative avec un cylindre perforé, et à partir duquel des feuilles individuelles du matériau textile non tissé, tissé ou tricoté avec des dépôts SAP uniformément espacés sont ultérieurement coupées, de préférence
- comprenant en outre, après l'étape de traitement thermique, une étape de découpe de feuilles individuelles de matériau textile non tissé, tissé ou tricoté avec des dépôts SAP uniformément espacés à partir du rouleau continu, et/ou
- le matériau textile non tissé, tissé ou tricoté, avant l'étape de dépôt, a une densité comprise de préférence entre 20 et 60 g/m$^2$, plus préférentiellement entre 20 et 30 g/m$^2$, et/ou
- après traitement thermique, le poids des dépôts par rapport au matériau textile est compris entre 200% et 600%, plus préférentiellement entre 400% et 600%, et/ou
- dans lequel les dépôts sont uniformément espacés sur le matériau textile non tissé, tissé ou tricoté, l'espacement étant de préférence compris entre 1 et 6 mm, de préférence environ 2 mm, et/ou
- dans lequel le matériau textile et le réseau de fibres sont en polypropylène, et/ou
- dans lequel les dépôts de SAP ont la forme de points circulaires ou de carrés sur le matériau textile, et/ou
- dans lequel le polymère synthétique est choisi parmi les homo- ou copolymères greffés linéaires ou ramifiés fabriqués à partir de monomères vinylacides tels que l'acide acrylique, l'anhydride maléique, l'anhydride itaconique et similaires, éventuellement en association avec d'autres types de monomères vinyliques qui ne contiennent pas nécessairement de fonctions acide carboxylique, et/ou
- dans lequel le polymère synthétique est un copolymère à base d'anhydride maléique et/ou d'acide maléique, de préférence un copolymère d'anhydride styrène-maléique (SMA), un copolymère d'isobutylène et d'anhydride maléique ou un copolymère d'éther méthylvinylique et d'acide maléique, et/ou
- dans lequel le polymère naturel est un biopolymère choisi parmi les polysaccharides tels que la cellulose, l'alginate, le dextrane, le chitosane et autres ; les polyesters et la lignine ; le guar ; l'amidon ; les phospholipides solubles dans l'eau tels que la lécithine ; les polypeptides ou les protéines tels que la gélatine, l'albumine, les protéines de soja, le collagène, le biopolymère collagénique, l'hydrolysat de collagène et la caséine.

**14.** Procédé de préparation d'une couche de fil plastique texturé avec des dépôts uniformément espacés d'un polymère naturel réticulé à un polymère synthétique dans le produit absorbant multicouche de la revendication 9, comprenant :

- déposer sur un fil de plastique texturé, qui a un réseau de surface de pores, des dépôts uniformément espacés d'une solution aqueuse composite polymère sous la forme d'un sirop pour l'utilisation dans l'amélioration de l'absorption de l'eau du fil de plastique texturé,

- la solution composite polymère comprenant un polymère synthétique ayant des monomères portant des groupes acide carboxylique ou anhydride d'acide carboxylique et un polymère naturel ayant des groupes amino et/ou hydroxyle capables de se réticuler aux groupes COOH du polymère synthétique, dans laquelle les polymères naturels et synthétiques dans la solution composite sont capables de subir une auto-réticulation dans des conditions contrôlées de température et de temps et en l'absence d'agent de réticulation non polymérique, dans lequel la concentration de la combinaison de polymères naturels et synthétiques est comprise entre 20 et 40 % en poids de la solution composite polymère, dans lequel le fil de plastique texturé présente une surface poreuse créée par des trous non pénétrants, des indentations, des dépressions, des bulles ouvertes et d'autres caractéristiques de rugosité formées par des méthodes de moussage, de gravure, de coupe ou de façonnage des vides, et présente, avant le dépôt, une densité comprise entre 20 et 150 g/m$^2$ ; et

- traiter thermiquement le fil plastique texturé présentant des dépôts uniformément espacés dans des conditions contrôlées de température et de durée, de sorte que les polymères naturels et synthétiques du dépôt subissent une auto-réticulation en l'absence d'agent de réticulation non polymère dans les dépôts pour former un réseau polymère réticulé SAP continu qui recouvre les parois des pores sous les dépôts SAP et interpénètre le réseau de pores agissant comme un échafaudage pour interconnecter le réseau polymère réticulé SAP avec le réseau de pores, environ 10 % ± 1 % de chacun de ces dépôts de SAP interpénètrent le réseau de pores et environ 90 % ± 1 % du reste de chacun de ces dépôts de SAP sont collés à la surface poreuse supérieure du fil plastique texturé, le poids des dépôts de SAP par rapport au fil plastique texturé étant compris entre 100 % et 600 % et les dépôts de SAP imprimés sur le fil plastique texturé en tant que substrat étant uniformément espacés d'un espacement compris entre 1 et 7 mm.

**15.** Le procédé de la revendication 14,

- dans lequel, dans l'étape de dépôt, les dépôts uniformément espacés sont déposés sur un rouleau continu de fil plastique texturé par sérigraphie rotative avec un cylindre perforé, et à partir duquel des feuilles individuelles du fil plastique texturé avec des dépôts SAP uniformément espacés sur celui-ci sont ensuite coupées, de préférence
- comprenant en outre, après l'étape de traitement thermique, une étape de découpe de feuilles individuelles de fil plastique texturé avec des dépôts SAP uniformément espacés à partir du rouleau continu, et/ou
- dans lequel, après traitement thermique, le poids des dépôts par rapport au fil en plastique est compris entre 200 % et 600 %, de préférence entre 400 % et 600 %, et/ou
- dans lequel les dépôts sont uniformément espacés sur le fil plastique texturé, l'espacement étant de préférence compris entre 1 et 6 mm, de préférence environ 2 mm, et/ou
- dans lequel le fil plastique texturé et le réseau de pores sont en polyéthylène, et/ou
- dans lequel les dépôts de SAP ont la forme de points circulaires ou de carrés sur le fil de plastique texturé, et/ou
- dans lequel le polymère synthétique est de préférence choisi parmi les homo- ou copolymères greffés linéaires ou ramifiés fabriqués à partir de monomères vinylacides tels que l'acide acrylique, l'anhydride maléique, l'anhydride itaconique et similaires, éventuellement en association avec d'autres types de monomères vinyliques qui ne contiennent pas nécessairement de fonctions acide carboxylique, et/ou
- dans lequel le polymère synthétique est un copolymère à base d'anhydride maléique et/ou d'acide maléique, de préférence un copolymère d'anhydride styrène-maléique (SMA), un copolymère d'isobutylène et d'anhydride maléique ou un copolymère d'éther méthylvinylique et d'acide maléique, et/ou dans lequel le polymère naturel est un biopolymère choisi parmi les polysaccharides tels que la cellulose, l'alginate, le dextrane, le chitosane, etc ; les polyesters et la lignine ; le guar ; l'amidon ; les phospholipides hydrosolubles tels que la lécithine ; les polypeptides ou les protéines tels que la gélatine, l'albumine, la protéine de soja, le collagène, le biopolymère collagénique, l'hydrolysat de collagène et la caséine.

Fig. 1

Fig. 2

Fig. 3

Fig.4

Fig. 5A

Fig 5B

Fig. 5C

FIG. 6A

liquid

DEPOSITS

FIG. 6B

liquid

Fig. 6

Fig. 7A

DEPOSITS

Fig. 7B

## REFERENCES CITED IN THE DESCRIPTION

**Patent documents cited in the description**

- US 4537590 A
- US 4540454 A
- US 4573988 A
- US 4676784 A
- US 5567478 A
- US 5962068 A
- US 6417425 B
- US 6645407 B
- US 2003120231 A
- WO 2019195271 A
- WO 2016090330 A
- US 4057521 A
- US 4861539 A
- US 4962172 A
- US 4963638 A
- US 5280079 A
- US 5413747 A
- US 2988539 A
- US 3393168 A
- US 3514419 A
- US 3926891 A
- US 3980663 A
- US 4155957 A
- US 5693707 A, Cheng
- US 6506696 B, Goldstein
- US 7205259 B, Soerens Dave Allen
- US 3983271 A
- US 4066584 A
- US 4320040 A
- US 4418163 A
- US 4731067 A
- US 4855179 A
- US 4880868 A
- US 4888238 A
- US 5698074 A
- US 5997791 A
- US 6150495 A
- US 6162541 A
- US 6241713 B
- US 6773746 B
- US 6824650 B
- US 7300965 B, Weerawarna
- US 5137537 A
- US 5183707 A
- US 5190563 A
- US 6689378 B, Sun
- US 4210489 A
- US 4242408 A
- US 7985819 B

**Non-patent literature cited in the description**

- **MARGARETH R.C. MARQUES et al.** Simulated Biological Fluids with Possible Application in Dissolution Testing. *Dissolution Technologies,* August 2011, vol. 1